# FASCICULE DE BREVET EUROPEEN

(11) **EP 0 623 138 B1**
(45) Date de publication et mention de la délivrance du brevet: **21.05.1997**
(21) Numéro de dépôt: 93914481.2
(22) Date de dépôt: 29.12.1992
(51) Int. Cl.: C07H 19/167, A61K 31/70

(54) **NOUVEAUX DERIVES DE L'ADENOSINE, LEURS PROCEDES DE PREPARATION, COMPOSITIONS PHARMACEUTIQUES LES CONTENANT**
Adenosinderivate, Verfahren zu deren Herstellung und pharmazeutische Mittel
NEW ADENOSINE DERIVATIVES, PREPARATION METHODS AND PHARMACEUTICAL COMPOSITIONS CONTAINING THEM

(30) Priorité: 08.01.1992 FR 9200138
(43) Date de publication de la demande: 09.11.1994
(73) Titulaire: LABORATOIRES UPSA, 47000 Agen (FR)
(72) Inventeur: BRU-MAGNIEZ, Nicole, F-75016 Paris (FR); GUNGOR, Timur, F-92500 Rueil-Malmaison (FR); TEULON, Jean-Marie, F-78170 La-Celle-Saint-Cloud (FR)
(74) Mandataire: Hubert, Philippe
(86) Numéro de dépôt international: FR9201241
(87) Numéro de publication internationale: WO9314102

(56) Documents cités:
- EP-A- 0 267 878
- WO-A-86/00310
- WO-A-88/03147
- WO-A-88/03148
- WO-A-92/05177
- FR-A- 2 154 527
- JOURNAL OF MEDICINAL CHEMISTRY. vol. 16, no. 4, Avril 1973, WASHINGTON US pages 358 - 64 K.KIKUGAWA ET AL. 'Platelet Aggregation Inhibitors. 4. N-Substituted Adenosines.' cité dans la demande

## Description

La présente invention concerne en tant que produits nouveaux, les dérivés d'adénosine de formule générale (I) ci-dessous et éventuellement leurs sels d'addition en particulier les sels d'addition pharmaceutiquement acceptables.

Les composés en question présentent un profil pharmacologique très intéressant dans la mesure où ils sont doués d'une part et particulièrement de propriétés antalgiques d'autre part de propriétés antihypertensives.

La présente invention concerne également le procédé de préparation des dits produits, les intermédiaires de synthèse et l'application de ces produits en thérapeutique.

Ces dérivés de l'adénosine sont caractérisés en ce qu'ils répondent à la formule générale (I) : 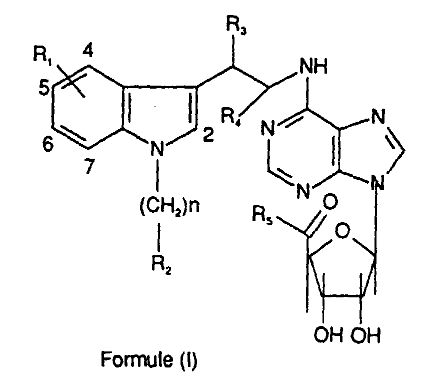 dans laquelle :
- R₁ représente un atome d'hydrogène, un atome d'halogène, un radical alkyle inférieur, un radical O-alkyle inférieur, un radical S-alkyle inférieur, ou un radical phényle et peut se trouver en position 2,4,5,6 ou 7 de l'indole ;
- n est un nombre entier de 0 à 4,
- R₂ représente un radical alkyle inférieur, un radical alcényle intérieur, un radical alcynyle inférieur, un radical cycloalkyle en C₃-C₇, un radical O-alkyle inférieur,
- un radical phényle ou naphtyle non substitué ou substitué par un à quatre substituants identiques ou différents choisis parmi un atome d'halogène, un groupement nitro, alkyle inférieur, O-alkyle inférieur, S- alkyle inférieur, NR₇R₈, R₇ et R₈ représentant l'atome d'hydrogène ou un radical alkyle inférieur,
- un radical hétérocyclique choisi parmi la pyridine, le thiophène non substitué ou substitué par un à quatre substituants identiques ou différents choisis parmi un atome d'halogène, un groupement nitro, alkyle inférieur, O-alkyle inférieur, S-alkyle inférieur,
- ou encore quand n est égal à 2,3 ou 4 un groupement -NR₉R₁₀, R₉ et R₁₀ représentant simultanément un radical alkyle inférieur ou formant ensemble avec l'atome d'azote auquel ils sont rattachés un hétérocycle choisi parmi la morpholine, la pipéridine, la pyrrolidine,
- R₃ et R₄ identiques ou différents représentent l'atome d'hydrogène ou un radical alkyle inférieur,
- R₅ représente un groupement NHR₁₁, R₁₁ étant un radical alkyle inférieur, un radical cycloalkyle en C₃-C₇, une chaîne alkyle inférieur possédant une fonction alcool, éther ou encore un groupement -(CH₂)ₙ-NR₉R₁₀, n, R₉ et R₁₀ étant définis comme précédemment.

Avantageusement, les dérivés conformes à l'invention sont les dérivés de formule (I) précitée dans laquelle :
- R₁ représente un atome d'hydrogène, un atome d'halogène, un radical alkyle inférieur, un radical O-alkyle inférieur, un radical S-alkyle inférieur, ou un radical phényle et peut se trouver en position 2 ou 5 de l'indole ;
- n est un nombre entier égal à 0, 1 ou 2 ;
- R₂ représente un radical alkyle inférieur, un radical alcényle inférieur, un radical alcynyle inférieur, un radical cycloalkyle en C₃-C₇, un radical O-alkyle inférieur ;
- un radical phényle ou naphtyle, non substitué ou substitué par un ou deux substituants identiques ou différents choisis parmi un atome d'halogène, un groupe nitro, alkyle inférieur, O-alkyle inférieur, -NR₇R₈, R₇ et R₈ représentant l'atome d'hydrogène ou un radical alkyle inférieur;
- un radical hétérocyclique choisi parmi la pyridine, le thiophène non substitué ou substitué par un atome d'halogène
- ou encore quand n = 2, un groupement -NR₉R₁₀, R₉ et R₁₀ représentant simultanément un radical alkyle inférieur ou formant ensemble avec l'atome d'azote auquel ils sont attachés un hétérocycle choisi parmi la morpholine, la pipéridine, la pyrrolidine ;
- R₃ et R₄, identiques ou différents représentent l'atome d'hydrogène ou un radical alkyle inférieur ;
- R₅ représente un groupement NHR₁₁, R₁₁ étant un radical alkyle inférieur, un radical cycloalkyle en C₃-C₇, une chaîne alkyle inférieure possédant une fonction alcool ou éther.

Dans la description et les revendications on entend par radical alkyle inférieur une chaîne hydrocarbonée ayant de 1 à 6 atomes de carbone, linéaire ou ramifiée. Un radical alkyle inférieur est par exemple, un radical méthyle, éthyle, propyle, isopropyle, butyle, isobutyle, tertiobutyle, pentyle, isopentyle, hexyle, isohexyle.

Dans la description et les revendications, on entend par radical alcényle inférieur une chaîne hydrocarbonée ayant de 1 à 6 atomes de carbone, linéaire ou ramifiée, possédant une double liaison comme par exemple un groupement éthényl ; et par radical alcynyle inférieur une chaîne hydrocarbonée ayant de 1 à 6 atomes de carbone, linéaire ou ramifiée, possédant une triple liaison comme par exemple un groupement éthynyl.

On entend par radical cycloalkyle en C₃-C₇ un radical cyclique saturé ; il s'agit de préférence d'un radical cyclopropyle, cyclobutyle, cyclopentyle, cyclohexyle, cycloheptyle.

On entend par halogène un atome de chlore, de brome, d'iode ou de fluor.

On entend par chaîne alkyle inférieure possédant une fonction alcool, une chaîne alkyle inférieure dont l'un des atomes d'hydrogène a été substitué par un groupement hydroxy . Une telle chaîne est par exemple la chaîne 1-hydroxy 2-méthyl propan-2-yl.

On entend par chaîne alkyle inférieure possédant une fonction éther une chaîne alkyle inférieure dont l'un des atomes d'hydrogène a été substitué par un groupement O-alkyle inférieur. Une telle chaîne est par exemple la chaîne 2-méthoxy éthyle.

Etant donné le potentiel thérapeutique de l'adénosine elle-même, de nombreux dérivés de ce nucléoside ont été décrits dans la littérature. On peut, par exemple, citer les documents suivants :
- Journal of Medicinal Chemistry 1973, vol. 16, n° 4, pages 358-64
- FR - 2 154 527
- EP - 0 267 878
- WO - 88/03 148
- WO - 88/03 147
- WO - 86/00 310
- WO - 92/05 177
- Biochemical Pharmacology 1974, vol. 23, pages 2283-89
- WO 86/00 310
- US 4,167,565
- EP 0232813
- US 5,023,244

Parmi ces nombreux documents, seuls essentiellement deux dérivés de l'indole en position 6 de l'adénosine ont été cités.

Ainsi :
l'article publié dans Journal of Medicinal Chemistry et le brevet FR 2154527 décrivent tous les deux le même produit :
la N-6-[(indolyl-3)-2 éthyle] adénosine (dérivé A) : 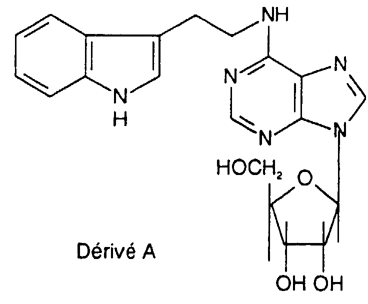 l'article du Biochemical Pharmacology décrit un dérivé de 5-méthoxy tryptamine, également cité dans le document FR 2154527 (dérivé B) :

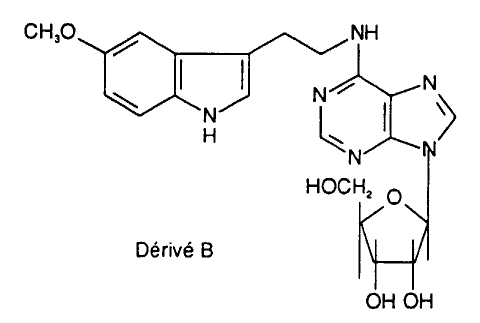

On peut noter que l'article du Journal of Medicinal Chemistry décrit pour le dérivé A une activité inhibitrice de l'aggrégation plaquettaire alors que le brevet français mentionne sans autre précision des effets sur le système nerveux central, sur la circulation et sur le coeur.
Biochemical Pharmacology précise que le dérivé B a une activité antilipolytique.

On peut remarquer que sur les composés décrits, d'une part les dérivés indoliques ne sont jamais substitués sur l'atome d'azote de l'indole et d'autre part le sucre de l'adénosine est intact.

Or la demanderesse a découvert que d'une façon surprenante et inattendue, la substitution de l'atome d'azote du cycle indolique combinée avec la transformation de l'alcool primaire du sucre en fonction amide conférait aux produits un profil pharmacologique particulièrement intéressant notamment dans le domaine antalgique.

Selon une variante de réalisation R₁ représente l'atome d'hydrogène.

Selon une autre variante de réalisation, R₁ représente un radical méthyle.

Selon une autre variante de réalisation, R₁ représente un radical méthoxy.

Selon une variante de réalisation n est un nombre égal à 0.

Selon une autre variante de réalisation, n est un nombre égal à 1.

Selon une autre variante de réalisation, n est un nombre égal à 2.

Selon une variante de réalisation, R₂ représente un radical méthoxy.

Selon une autre variante de réalisation, R₂ représente un radical cyclopentane.

Selon une autre variante de réalisation, R₂ représente un radical isopropyle.

Selon une autre variante de réalisation, R₂ représente un radical 2,5-diméthyl phényle.

Selon une autre variante de réalisation, R₂ représente un radical pipéridine.

Selon une variante de réalisation, R₃ représente l'atome d'hydrogène.

Selon une variante de réalisation, R_{4 r}eprésente l'atome d'hydrogène.

Selon une autre variante de réalisation, R₄ représente un radical méthyle.

Selon une variante de réalisation, R₅ représente un radical N-cyclopropyl amine.

Les composés de l'invention particulièrement préférés sont ceux qui sont choisis parmi les dérivés de formule :

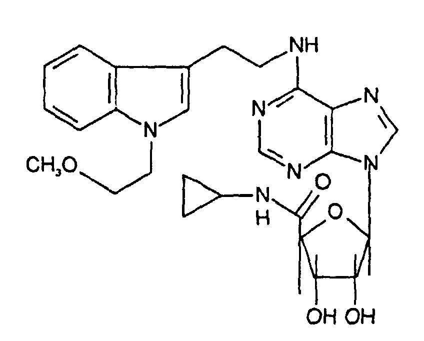

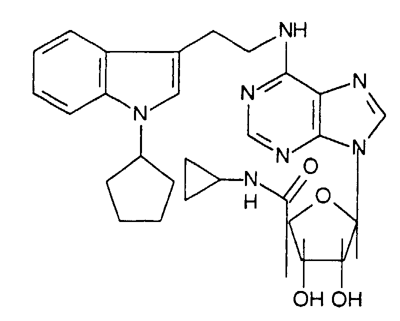

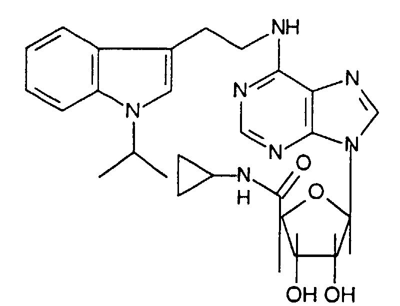

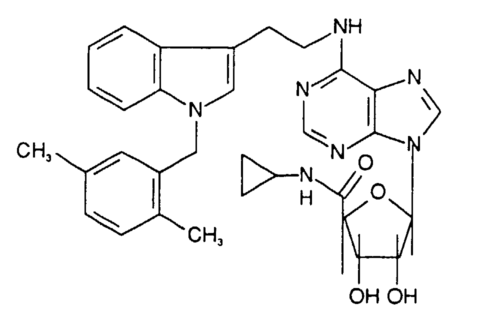

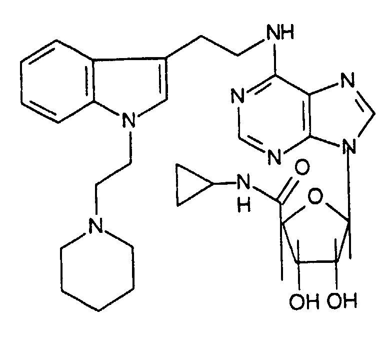

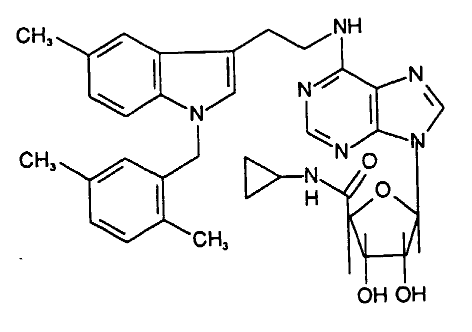

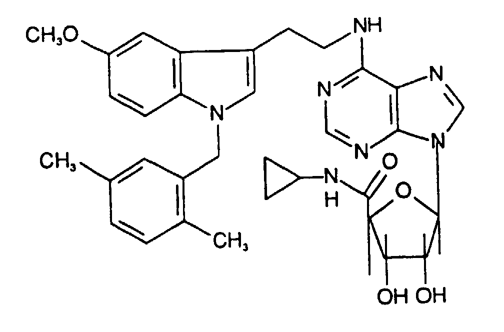

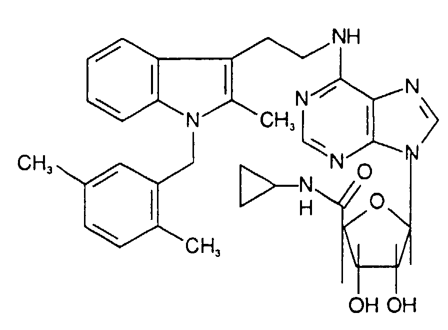

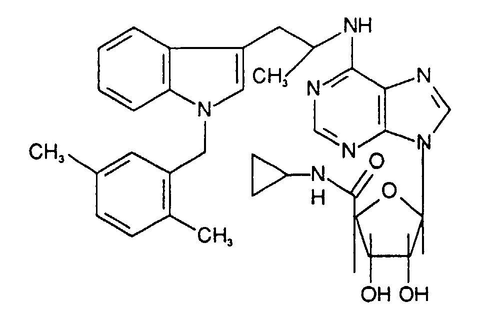

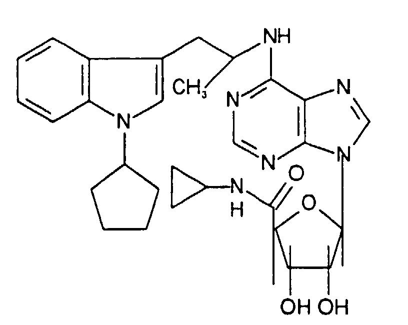

Selon l'invention, les composés de formule (I) pourront être synthétisés de la façon suivante :
l'action d'une amine de formule (II) : 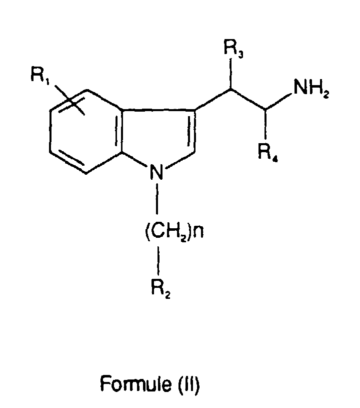 dans laquelle R₁, R₂, R₃, R₄ et n sont définis comme ci-dessus, sur les 6-halopurines ribosides de formule (III) 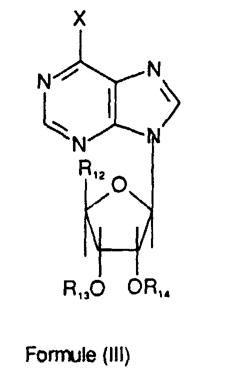 dans laquelle X représente un atome d'halogène, chlore ou brome de préférence, R₁₂ représente le groupement COR₅, R₅ étant défini comme précédemment ou le groupement CH₂OH, R₁₃ et R₁₄ représentent des groupements protecteurs de la fonction alcool comme un acétyl, un benzoyl ou un benzyl par exemple ou peuvent former ensemble un autre groupement protecteur de structure dioxolane par exemple,
dans un solvant comme un alcool par exemple ou un solvant aprotique comme le diméthyle formamide, en présence d'une base, comme la triéthylamine, la pyridine ou d'un carbonate de sodium, potassium ou calcium ou encore en présence de deux équivalents d'amine de formule (Il) à une température comprise entre 20° et 140°C conduira aux composés de formule (IV) 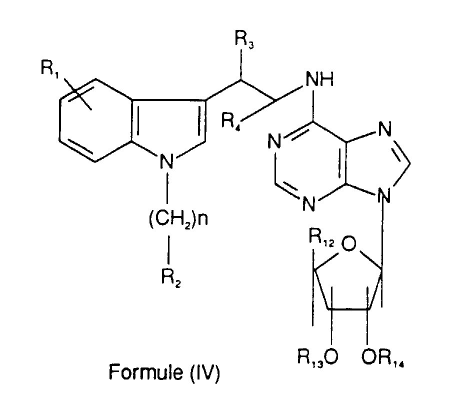 dans laquelle R₁, R₂, R₃, R₄ R₁₂, R₁₃ , R₁₄ et n sont définis comme ci-dessus.

Lorsque le dérivé indole amine de formule (II) présente un centre d'asymétrie, les composés doivent être considérés soit sous la forme racémique, soit sous la forme optiquement active. On prendra soin, lorsqu'on désire obtenir le dérivé optiquement actif de séparer les stéréoisomères au niveau de l'amine indolique, avant le couplage sur les 6-halopurines ribosides de formule (III), par des méthodes classiques de séparation des isomères optiques connues de l'homme de l'art, par exemple par recristallisation des sels formés avec un acide tartrique optiquement actif. Après séparation des tartrates optiquement actifs, la base optiquement active libérée de son acide tartrique sera couplée avec les 6-halopurines ribosides de formule (III).

Dans le cas où le radical R₁₂ représente le groupement CH₂OH, on pourra l'oxyder avec de l'anhydride chromique selon la méthode décrite par :
R.R. SCHMIDT et H.J. FRITZ Chem. Ber. 1970, 103, 1867
ou par le permanganate de potassium en présence d'ammoniaque selon la méthode décrite par :
P.J. HARPER et A. HAMPTON J. Org. Chem. 1970, 35 n° 5, 1688
l'acide ribouronique ainsi obtenu étant ensuite converti en chlorure d'acide par action du chlorure de thionyle, par exemple, puis en amide par action d'une amine selon les méthodes connues de l'homme de l'art. La déprotection des alcools secondaires OR₁₃, OR₁₄ pourra être effectuée selon différentes méthodes, par exemple en milieu basique comme l'alcool ammoniacal ou en milieu acide comme une solution d'acide chlorhydrique normale ou d'acide formique, à des températures variant de 0° à 70°C selon la nature des groupements protecteurs.

Ces suites de réactions permettent de transformer les dérivés de formule (IV) en dérivés de formule (I).

Les composés de formule (II) pourront être obtenus :
- soit par alkylation directe de dérivés indole éthylamine de formule (V) (qui sont commerciaux ou dont la synthèse est décrite dans la littérature
   P.L. JULIAN, E.W. MEYER et H.C. PRINTY, Heterocyclic compounds John Wiley and Sons, Inc. New York, 1952, Vol. 3, chapitre 1, p 51-57
   J. HARLEY-MASON et A.H. JACKSON J. Chem. Soc. 1954, 1165) 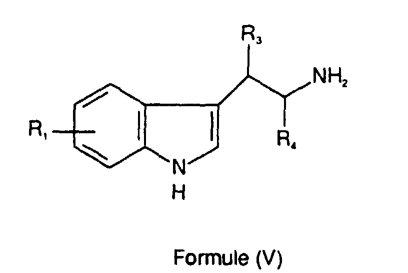
   dans laquelle R₁, R₃, R₄ sont définis comme ci-dessus, par des dérivés de formule (Vl)

      R₂-(CH₂)n-Y Formule (VI)
   dans laquelle R₂ et n sont définis comme ci-dessus, Y représentant un atome d'halogène de préférence chlore ou brome,
      en présence d'un agent métallant comme l'hydrure de sodium ou de lithium ou d'un alcoolate de sodium ou de potassium, dans un solvant organique comme un alcool ou comme le diméthyl formamide ou la N-méthyl pyrrolidone à des températures comprises entre 0° et 60°C ;
- soit par alkylation de formyl-3 indole de formule (VII) 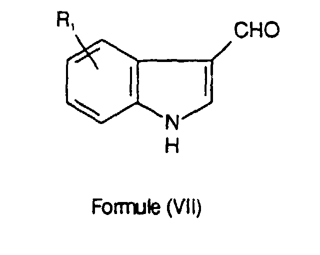
   dans laquelle R₁ est défini comme ci-dessus, par des dérivés de formule (VI) précités en présence d'un agent de métallation comme l'hydrure de sodium ou de lithium ou d'un alcoolate de sodium ou de potassium ou d'un carbonate de sodium ou de potassium, dans un solvant organique comme un alcool ou le diméthyl formamide pour conduire aux dérivés de formule (VIII) 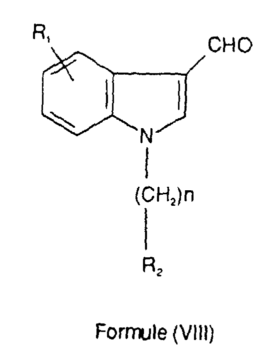
   dans laquelle R₁, R₂ et n sont définis comme ci-dessus ;
   ces dérivés étant ensuite mis en réaction avec le nitroalcane approprié en présence d'acétate d'ammonium pour conduire aux nitro vinyl indoles de formule (IX) : 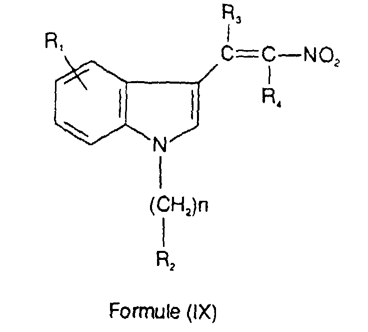
   dans laquelle R₁, R₂, R₃, R₄ et n sont définis comme ci-dessus,
   ces dérivés étant alors réduits par hydrogénation catalytique en présence de nickel de Raney ou par l'hydrure double d'aluminium et de lithium pour donner les composés de formule (II).

D'autres voies de synthèse de dérivés indole éthylamines sont communément décrites dans la littérature et peuvent être utilisées. On peut citer par exemple la voie de synthèse qui consiste à faire réagir le chlorure d'oxalyle sur l'indole approprié selon la référence

M.E. SPEETER et W.C. ANTHONY J. Am. Chem. Soc. 1954, 76, 6208 suivi d'une amidation et réduction de la fonction amide par l'hydrure double d'aluminium et de lithium.

Les formyl-3 indoles de formule (VII) utilisés dans ces synthèses sont commerciaux ou connus de l'homme de l'art, par exemple par la référence :
Organic Syntheses Coll. Vol. IV 539 ;
ou peuvent être obtenus par des méthodes décrites dans la littérature, comme par exemple dans :

Organic Syntheses Coll. Vol. IV 542.

Les 6-halopurines de formule (III) sont préparés à partir de l'inosine selon des méthodes décrites dans la littérature :
R.R. SCHMIDT et H. J. FRITZ, Chem. Ber. 1970, 103, 1867
H.M. KISSMAN et M.J. WEISS, J. Org. Chem. 1956, 21, 1053
B.R. BAKER ; K. HEWSON ; H.J. THOMAS et J.A. JOHNSON JR J. Org. Chem. 1957, 22, 954
J. ZEMLICKA et F. SORM, Coll. Czech. Chem. Commun. 1965, 30, (6), 1880.

Les composés de formule (I) tels que définis ci-dessus ainsi que leurs sels d'addition, en particulier les sels d'addition pharmaceutiquement acceptables sont doués d'une bonne affinité pour les récepteurs à l'adénosine. Cette affinité leur confère une bonne activité antalgique mais également des propriétés antihypertensives.

Ces propriétés justifient l'application des dérivés de formule (I) en thérapeutique et l'invention a également pour objet, à titre de médicaments, les produits tels que définis par la formule (I) ci-dessus, ainsi que leurs sels d'addition, en particulier les sels d'addition pharmaceutiquement acceptables.

Ainsi, l'invention couvre également une composition pharmaceutique, caractérisée en ce qu'elle comprend une quantité pharmaceutiquement efficace d'au moins un composé de formule (I) tel que précédemment défini, ou un de ses sels d'addition pharmaceutiquement acceptables, éventuellement incorporé dans un excipient, véhicule ou support pharmaceutiquement acceptable.

Ces compositions peuvent être administrées par voie buccale, rectale, par voie parentérale, par voie transdermique ou par voie occulaire.

Ces compositions peuvent être solides ou liquides et se présenter sous les formes pharmaceutiques couramment utilisées en médecine humaine comme, par exemple, les comprimés simples ou dragéifiés, les gélules, les granulés, les suppositoires, les préparations injectables, les systèmes transdermiques et les collyres. Elles sont préparées selon les méthodes usuelles. Le principe actif, constitué par une quantité pharmaceutiquement efficace d'au moins un composé de formule (I) défini comme ci-dessus ou un de ses sels d'addition pharmaceutiquement acceptable, peut y être incorporé à des excipients habituellement employés dans ces compositions pharmaceutiques, tels que le talc, la gomme arabique, le lactose, l'amidon, le stéarate de magnésium, la polyvidone, les dérivés de la cellulose, le beurre de cacao, les glycérides semi-synthétiques, les véhicules aqueux ou non, les corps gras d'origine animale ou végétale, les glycols, les divers agents mouillants, dispersants ou émulsifiants, les gels de silicone, certains polymères ou copolymères, les conservateurs, arômes et colorants.

L'invention couvre encore une composition pharmaceutique à activité antalgique permettant notamment de traiter favorablement la douleur, caractérisée en ce qu'elle comprend une quantité pharmaceutiquement efficace d'au moins un composé de formule (I) précitée ou un de ses sels d'addition pharmaceutiquement acceptable, éventuellement incorporé dans un excipient, véhicule ou support pharmaceutiquement acceptable.

L'invention couvre encore une composition pharmaceutique à activité antihypertensive permettant de traiter favorablement l'hypertension caractérisée en ce qu'elle comprend une quantité pharmaceutiquement efficace d'au moins un composé de formule (I) précitée ou un de ses sels d'addition pharmaceutiquement acceptable, éventuellement incorporé dans un excipient, véhicule ou support pharmaceutiquement acceptable.

L'invention couvre encore un procédé de préparation d'une composition pharmaceutique caractérisé en ce que l'on incorpore une quantité pharmaceutiquement efficace d'au moins un composé de formule (I) telle que précédemment définie ou un de ses sels d'addition pharmaceutiquement acceptables dans un excipient, véhicule ou support pharmaceutiquement acceptable. Selon un mode de réalisation, on prépare une composition pharmaceutique à activité antalgique permettant notamment de traiter favorablement la douleur ; selon un autre mode de réalisation, on prépare une composition pharmaceutique à activité antihypertensive permettant notamment de traiter favorablement l'hypertension.

Selon une autre variante de réalisation, on prépare une composition pharmaceutique formulée sous forme de gélules ou de comprimés dosés de 5 à 300 mg ou sous forme de préparations injectables dosées de 0,1 mg à 100 mg. On pourra également utiliser des formulations sous forme de suppositoires, pommades, crèmes, gels ou des préparations en aérosols.

L'invention couvre encore un procédé de traitement thérapeutique des mammifères caractérisé en ce qu'on administre à ce mammifère une quantité thérapeutiquement efficace d'au moins un composé de formule (I) telle que précédemment définie, ou un de ses sels d'additions pharmaceutiquement acceptable. Selon une variante de réalisation de ce procédé de traitement, le composé de formule (I), soit seul, soit en association avec un excipient pharmaceutiquement acceptable, est formulé en gélules ou en comprimés dosés de 5 mg à 300 mg pour l'administration par voie orale, ou sous forme de préparations injectables dosées de 0,1 à 100 mg ou encore sous forme de suppositoires, pommades, crèmes, gels ou de préparations en aérosols.

En thérapeutique humaine et animale, les composés de formule (I) et leurs sels peuvent être administrés seuls ou en association avec un excipient physiologiquement acceptable sous forme quelconque, en particulier par voie orale sous forme de gélules ou de comprimés ou par voie parentérale sous forme de soluté injectable. D'autres formes d'administration comme suppositoires, pommades, crèmes, gels ou des préparations en aérosols peuvent être envisagées.

Comme il ressortira clairement des essais de pharmacologie donnés en fin de description les composés selon l'invention peuvent être administrés en thérapeutique humaine dans les indications précitées par voie orale sous forme de comprimés ou gélules dosés de 5 mg à 300 mg ou par voie parentérale sous forme de préparations injectables dosées de 0,1 mg à 100 mg en une ou plusieurs prises journalières pour un adulte de poids moyen 60 à 70 kg.

En thérapeutique animale la dose journalière utilisable devrait habituellement se situer de 0,1 à 50 mg par kg par voie orale et de 0,01 à 1 mg par kg par voie intraveineuse.

D'autres caractéristiques et avantages de l'invention seront mieux compris à la lecture qui va suivre de quelques exemples nullement limitatifs, mais donnés à titre d'illustration.

### Exemple 1: β-D-Ribofuranuronamide, 1-(6-chloro-9H-purin-9-yl)-N-cyclo propyl-1-deoxy-2,3-O-(1-methylethylidene)

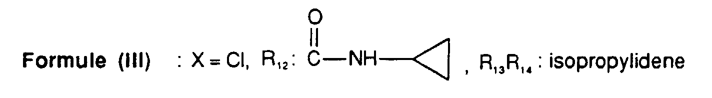

20 g de 2',3'-O-isopropylidène 6 chloro purine 5' uronic acide préparé selon SCHMIDT R.R. et FRITZ H.J. Chem. Ber. 1970, 103(6) 1867-71 dans 500 ml de CHCl₃ anhydre stabilisé à l'amylène sont portés au reflux pendant 5 h en présence de 86 ml de SOCl₂ et 10 ml de DMF anhydre.

Le SOCl₂ en excès ainsi que les solvants sont distillés. Le résidu est repris avec 200 ml de chloroforme anhydre et rajouté goutte à goutte sous azote à un mélange de 150 ml de CHCl₃ et 41 ml de cyclopropylamine préalablement refroidit à 5°C. La température du mélange réactionnel est maintenue inférieure à 10°C lors de l'addition du chlorure d'acide.

Laisser agir 30 mn supplémentaires puis laver 3 fois avec une solution de HCI dilué puis avec une solution de bicarbonate de sodium. Un ultime lavage à l'eau permet après séchage et évaporation du solvant d'obtenir 26.3 g d'une huile brune.

La purification par chromatographie sur gel de silice (éluant CH₂Cl₂ 90 % / Acétone 10 %) conduit à 15.7 g de β-D-Ribofuranuronamide, 1-(6-chloro-9H-purin-9-yl)-N-cyclo propyl-1-deoxy-2,3-O-(1-methylethylidene) sous forme de solide amorphe.

Selon le mode opératoire de l'exemple 1 mais en utilisant les amines appropriées les composés des exemples 2 à 4 ont été préparés :

### Exemple 2 : β-D-Ribofuranuronamide, 1-(6-chloro-9H-purin-9-yl)-1-deoxy-N-ethyl-2,3-O-(1-methylethylidene)

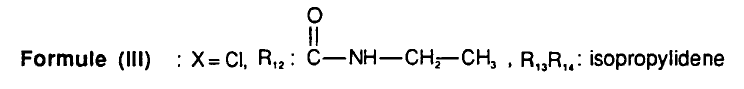

Huile jaunâtre purifiée par chromatographie sur gel de silice (éluant chloroforme 95 % / méthanol 5 %) pour donner un solide de point de fusion 91°C.

### Exemple 3 : β-D-Ribofuranuronamide, 1-(6-chloro-9H-purin-9-yl)-1-deoxy-N-(1-hydroxy-2-méthylpropan-2-yl)-2,3-O-(1-methyl ethylidene)

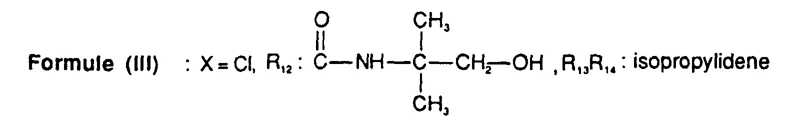

Huile brune purifiée par chromatographie sur gel de silice (éluant chloroforme 90 % / méthanol 10 %).

### Exemple 4 : β-D-Ribofuranuronamide, 1-(6-chlore-9H-purin-9-yl)-1-deoxy-N-isopropyl-2,3-O-(1-methylethylidene)

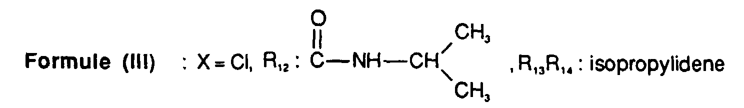

Huile orangée purifiée par chromatographie sur gel de silice (éluant CHCl₃ 90 % /Acétone 10 %).

### Exemple 5 : 1-(4-chlorobenzyl) 3-formyl indole

**Formule (VIII) :** R₁ = H, n = 1, R₂ = 4-chlorophényl

58 g de formyl-3 indole, 55.9 g de K₂CO₃ et 70.9 g de p-chloro chlorure de benzyle en solution dans 200 ml de DMF sont portés au reflux pendant 2 h. Après refroidissement le mélange est coulé dans 2 l d'eau. Triturer. Le précipité obtenu est filtré, lavé à l'eau puis repris à l'isopropanol, essoré, tassé, lavé au pentane pour donner 120 g d'un solide crème.

Une purification par recristallisation dans l'éthanol fournit 84.4 g de 1-(4-chlorobenzyl) 3-formyl indole de point de fusion 122°C.

Selon le procédé de l'exemple 5, les composés suivants des exemples 6 à 16 ont été réalisés.

### Exemple 6: 1-benzyl 3-formyl indole

**Formule (VIII) :** R₁ = H, n = 1, R₂ = phényl

Recristallisation dans de l'éthanol.
Point de fusion : 111°C. (littérature : 113-114°C - A. KALIR et S. SZARA J. Med. Chem. (1966) Vol. 9 p.793)

### Exemple 7 : 1-(2,6-dichloro benzyl) 3-formyl indole

**Formule (VIII) :** R₁ = H, n = 1, R₂ = (2,6-dichlorophényl)

Recristallisation dans le 2-méthoxy éthanol.
Point de fusion : 160°C.

### Exemple 8 : 1-(1-naphtylméthyl) 3- formyl indole

**Formule (VIII) :** R₁ = H, n = 1, R₂ = naphtyl

Solide brut utilisé tel quel à l'étape suivante.

### Exemple 9 : 3-formyl 1-(3-pyridyl) indole

**Formule (VIII) :** R₁ = H, n = 1, R₂ = 3-pyridyl

Purification par chromatographie sur gel de silice (éluant CHCl₃ 95 % / Méthanol 5 %)
Point de fusion : 88°C.

### Exemple 10: 1-(4-méthyl benzyl) 3-formyl indole

Formule (VIII) :R₁ = H, n = 1, R₂ = 4-méthyl phényl

Solide brut utilisé tel quel à l'étape suivante.
Point de fusion 118°C.

### Exemple 11 : 1-(3,4-diméthyl benzyl) 3-formyl indole

**Formule (VIII) :** R₁ = H, n = 1, R₂ = 3,4-diméthylphényl

Huile brune utilisée telle quelle à l'étape suivante.

### Exemple 12 : 1-(2,5-diméthyl benzyl) 3-formyl indole

**Formule (VIII) :** R₁ = H, n = 1, R₂ = 2,5-diméthyl phényl

Solide brut utilisé tel quel à l'étape suivante.
Point de fusion : 139°C.

### Exemple 13 : 1-(2-méthoxy éthyl) 3-formyl indole

**Formule (VIII) :** R₁ = H, n = 2, R₂ = méthoxy

Huile brune utilisée telle quelle à l'étape suivante.

### Exemple 14 : 1-(cyclopentyl) 3-formyl indole

**Formule (VIII) :** R₁ = H, n = 0, R₂ = cyclopentyl

Huile brune purifiée par chromatographie sur gel de silice (éluant chloroforme 90 % / méthanol 10 %).

### Exemple 15 : 3-formyl 1-isopropyl indole

**Formule (VIII) :** R₁ = H, n = 0, R₂ = isopropyl

Huile brune utilisée telle quelle à l'étape suivante.

### Exemple 16 : 3-formyl 1-(2-N-morpholinoethyl) indole

**Formule (VIII) :** R₁ = H, n = 2, R₂ = N-morpholino

Solide utilisé tel quel à l'étape suivante.
Point de fusion : 80°C.

### Exemple 17 : 1-(4-chlorobenzyl) 3-(2-nitrovinyl) indole

**Formule (IX) :** R₁ = H, n = 1, R₂ = 4-chlorophényl, R₃ = R₄ = H

On porte au reflux pendant 30 mn, 80.9 g de 1-(4-chlorobenzyl) 3-formyl indole préparé à l'exemple 5, 18 g d'acétate d'ammonium et 300 ml de nitrométhane.

Un précipité orange apparaît après refroidissement. Filtrer, laver à l'eau puis à l'isopropanol et à l'hexane pour obtenir 81.1 g de cristaux oranges de 1-(4-chloro benzyl) 3-(2-nitrovinyl) indole.

Point de fusion : 178°C.

Selon le mode opératoire de l'exemple 17, les nitro vinyl indoles des exemples 18 à 28 ont été préparés.

### Exemple 18 : 1-benzyl 3-(2-nitrovinyl) indole

**Formule (IX) :** R₁ = H, n = 1, R₂ = phényl, R₃ = R₄ = H

Point de fusion : 130°C.

### Exemple 19 : 1-(2,6-dichlorobenzyl) 3-(2-nitrovinyl) indole

**Formule (IX) :** R₁ = H, n = 1, R₂ = 2,6 dichlorophényl, R₃ = R₄ = H

Point de fusion : 170°C.

### Exemple 20 : 1-(naphtyl methyl) 3-(2-nitrovinyl) indole

**Formule (IX) :** R₁ = H, n = 1, R₂ = naphtyl, R₃ = R₄ = H

Point de fusion : 196°C.

### Exemple 21 : 1-(3-pyridyl methyl) 3-(2-nitrovinyl) indole

**Formule (IX) :** R₁ = H, n = 1, R₂ = 3-pyridyl, R₃ = R₄ = H

Point de fusion : 165-170°C.

### Exemple 22 : 1-(4-methyl benzyl) 3-(2-nitrovinyl) indole

**Formule (IX) :** R₁ = H, n = 1, R₂ = 4-methyl phenyl, R₃ = R₄ = H

Huile orangée purifiée par chromatographie sur gel de silice (éluant chloroforme 95 % / isopropylamine 5 %).

### Exemple 23 : 1-(3,4-dimethyl benzyl) 3-(2-nitrovinyl) indole

**Formule (IX) :** R₁ = H, n = 1, R₂ = 3,4-dimethyl phenyl, R₃ = R₄ = H

Point de fusion : 135°C.

### Exemple 24 : 1-(2,5-dimethyl benzyl) 3-nitrovinyl indole

**Formule (IX) :** R₁ = H, n = 1, R₂ = 2,5-dimethyl phenyl, R₃ = R₄ = H

Point de fusion: 145°C.

### Exemple 25: 1-(2-methoxy ethyl) 3-(2-nitrovinyl) indole

**Formule (IX) :** R₁ = H, n = 2, R₂ = methoxy, R₃ = R₄ = H

Point de fusion : 132°C.

### Exemple 26 : 1-cyclopentyl 3-(2-nitrovinyl) indole

**Formule (IX) :** R₁ = H, n = 0, R₂ =cyclopentyl, R₃ = R₄ = H

Huile orangée purifiée par chromatographie sur gel de silice. Eluant dichlorométhane.

### Exemple 27 : 1-isopropyl 3-(2-nitrovinyl) indole

**Formule (IX) :** R₁ = H, n = 0, R₂ = isopropyl, R₃ = R₄ = H

Huile orangée utilisée telle quelle à l'étape suivante.

### Exemple 28 : 1-(2-N-morpholino ethyl) 3-(2-nitrovinyl) indole

**Formule (IX) :** R₁ = H, n = 2, R₂ = N-morpholino, R₃ = R₄ = H

Point de fusion : 114°C.

### Exemple 29 : 1-(4-chlorobenzyl) 3-(2-aminoethyl) indole

**Formule (II) :** R₁ = H, n = 1, R₂ = 4-chlorophenyl, R₃ = R₄ = H

Dans 500 ml de THF anhydre, ajouter par petites portions 52.5 g de Li AIH₄. Laisser remonter la température jusqu'à 50°C. Sans refroidir la solution, introduire goutte à goutte 78.2 g de 1-(4-chlorobenzyl) 3-(2-nitrovinyl) indole préparé à l'exemple 17 en solution dans 1000 ml de THF anhydre.

Chauffer 1 h 30 au reflux . Refroidir. Introduire goutte à goutte une solution aqueuse saturée de Na₂SO₄ . Filtrer sur célite 545. Décanter, concentrer la phase organique pour obtenir une huile orangée.

Le composé est purifié d'abord par distillation (température d'ébullition 180-188°C sous 0,1 mm de mercure) puis par recristallisation du chlorhydrate dans l'éthanol pour obtenir 38.1 g de chlorhydrate de 1-(4-chlorobenzyl) 3-(2-aminoethyl) indole.
Point de fusion de la base : 87°C.
Point de fusion du chlorhydrate : 212°C.

### Exemple 30 : 1-(4-chlorobenzyl) 3-(2-aminoethyl) indole

**Formule (Il) :** R₁ = H, n = 1, R₂ = 4-chlorophenyl, R₃ = R₄ = H

On dissout 10 g de 3-aminoethyl indole dans 50 cm³ de DMF. On introduit alors 5.6 g de NaH (60 %).

Agiter 30 mn à température ambiante.

Une solution de 11.2 g de p-chloro chlorure de benzyle dans 10 ml de DMF est introduite goutte à goutte. Chauffer à 55°C pendant 2 h. Refroidir. Filtrer l'insoluble . Concentrer sous vide, reprendre au chlorure de méthylène et laver à l'eau. Sécher, concentrer la phase organique pour obtenir 20.4 g d'une huile brune.

Une purification par chromatographie sur gel de silice (éluant CHCl₃ 95 % / isopropylamine 5 %) conduit à 9.7 g de 1-(4-chlorobenzyl) 3-(2-aminoethyl) indole.

Point de fusion du chlorhydrate : 214°C.

Selon l'un des modes opératoires des exemples 29 ou 30 les composés suivants des exemples 31 à 45 ont été préparés.

### Exemple 31 : 1-benzyl 3-(2-amino ethyl) indole

**Formule (II) :** R₁ = H, n = 1, R₂ = phenyl, R₃ = R₄ = H

Chlorhydrate, purifié par recristallisation dans l'isopropanol.
Point de fusion : 176-178°C.

### Exemple 32 : 1-(2,6-dichlorobenzyl) 3-(2 aminoéthyl) indole

**Formule (II) :** R₁ = H, n = 1, R₂ = 2,6-dichlorophényl, R₃ = R₄ = H

Point de fusion : 68°C.

### Exemple 33 : 1-(naphtyl methyl) 3-(2-aminoethyl) indole

**Formule (II) :** R₁ = H, n = 1, R₂ = naphtyl, R₃ = R₄ = H

Huile orangée, purifiée par chromatographie sur gel de silice (éluant chloroforme 95 % / isopropylamine 5 %).

### Exemple 34 : 1-(3-pyridyl methyl) 3-(2-aminoethyl) indole

**Formule (II) :** R₁ = H, n = 1, R₂ = 3-pyridyl, R₃ = R₄ = H

Huile purifiée par chromatographie sur gel de silice. (Eluant chloroforme 95 % / isopropylamine 5 %).

### Exemple 35 : 1-(4-methyl benzyl) 3-(2-aminoethyl) indole

**Formule (Il) :** R₁ = H, n = 1, R₂ = 4-methylphenyl, R₃ = R₄ = H

Huile orangée, purifiée par chromatographie sur gel de silice (éluant : chloroforme 95 % / isopropylamine 5 %).

### Exemple 36 : 1-(3,4-dimethylbenzyl) 3-(2-aminoethyl) indole

**Formule (Il) :** R₁ = H, n = 1, R₂ = 3,4-dimethylphenyl, R₃ = R₄ =H

Huile incolore purifiée par chromatographie sur gel de silice (éluant : dichloro methane 95 % / isopropylamine 5 %).

### Exemple 37 : 1-(2,5-dimethylbenzyl) 3-(2-aminoethyl) indole

**Formule (Il) :** R₁ = H, n = 1, R₂ = 2,5-dimethyl phenyl, R₃ = R₄ = H

Huile orangée purifiée par chromatographie sur gel de silice (éluant : dichloro methane 95 % / isopropylamine 5 %).

### Exemple 38 : 1-(2-methoxyethyl) 3-(2-aminoethyl) indole

**Formule (II) :** R₁ = H, n = 2, R₂ = methoxy, R₃ = R₄ = H

Huile orangée purifiée par chromatographie sur gel de silice (éluant : chloroforme 90 % / isopropylamine 10 %).

### Exemple 39: 1-cyclopentyl 3-(2-aminoethyl) indole

**Formule (II) :** R₁ = H, n = 0, R₂ = cyclopentyl, R₃ = R₄ = H

Huile jaunâtre purifiée par chromatographie sur gel de silice (éluant : chloroforme 95 % / isopropylamine 5 %).

### Exemple 40: 1-isopropyl 3-(2-aminoethyl) indole

**Formule (II) :** R₁ = H, n = 0, R₂ = isopropyl, R₃ = R₄ = H

Huile orangée purifiée par chromatographie sur gel de silice (éluant : chloroforme 95 % / isopropylamine 5 %).

### Exemple 41 : 1-(2-N,N-dimethylaminoethyl) 3-(2-aminoethyl) indole

**Formule (II) :** R₁ = H, n = 2, R₂ = N,N dimethyl amino, R₃ = R₄ = H

Huile orangée purifiée par chromatographie sur gel de silice (éluant : chloroforme 95 % / isopropylamine 5 %).

### Exemple 42 : 1-(2-N-morpholinoethyl) 3-(2-aminoethyl) indole

**Formule (II) :** R₁ = H, n = 2, R₂ = N-morpholino, R₃ = R₄ = H

Huile orangée purifiée par chromatographie sur gel de silice (éluant : chloroforme 95 % / isopropylamine 5 %).

### Exemple 43 : 1-(2-N-piperidinoethyl) 3-(2-aminoethyl) indole

**Formule (II) :** R₁ = H, n = 2, R₂ = N-piperidino, R₃ = R₄ = H

Huile orangée purifiée par chromatographie sur gel de silice (éluant : dichloromethane 95 % / isopropylamine 5 %).

### Exemple 44 : 1-(N-pyrrolidinoethyl) 3-(2-aminoethyl) indole

**Formule (II) :** R₁ = H, n = 2, R₂ = N-pyrrolidino, R₃ = R₄ = H

Huile orangée purifiée par chromatographie sur gel de silice (éluant : dichloromethane 95 % / isopropylamine 5 %).

### Exemple 45 : 1-(3,4 dichloro benzyl) 3-(2-aminoethyl) indole

**Formule (II) :** R₁ = H, n = 1, R₂ = 3,4-dichlorophenyl, R₃ = R₄ = H

Point de fusion : 196°C.

### Example 46 : β-D-Ribofuranuronamide, 1-[6-[[2-[1-(4-chlorobenzyl) indol-3-yl]ethyl] amino]-9H-purin-9-yl]-N-cyclopropyl 1-deoxy-2,3-O-(1- methylethylidene)

**Formule (IV) :** R₁ = H, n = 1, R₂ = 4-chlorophenyl, R₃ = R₄ = H

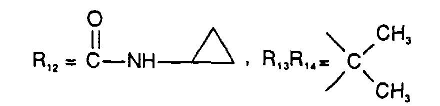

Sous un courant d'azote, on met en suspension dans 100 ml d'éthanol, 49 g de chlorhydrate de 1-(4-chlorobenzyl) 3-(2-aminoethyl) indole préparés selon l'une des procédures des exemples 29 ou 30. Neutraliser avec 5.1 ml de triéthylamine puis rajouter 4.1 g de β-D-Ribofuranuronamide, 1-(6-chloro-9H-purin-9-yl)- N-cyclopropyl-1-deoxy-2,3-O-(1-methylethylidene) préparé à l'exemple 1.

L'ensemble est porté au reflux pendant 7 h. Laisser reposer une nuit. Evaporer le solvant. Reprendre au chloroforme et laver à l'eau, sécher et concentrer. Le solide obtenu est chromatographié sur gel de silice (éluant chloroforme 90 % / méthanol 10 %) pour donner 7.2 g d'un solide amorphe.

Selon le mode opératoire de l'exemple 46 et en utilisant l'uronamide de l'exemple 1 les dérivés des exemples 47 à 59 ont été préparés sous forme de solides amorphes.

### Exemple 47 : β-D-Ribofuranuronamide, N-cyclopropyl-1-deoxy-1-[6-[[2-[1-(2-methoxy ethyl) indol-3-yl] ethyl] amino]-9H-purin-9-yl]-2,3-O-(1-methylethylidene)

**Formule (IV) :** R₁ = H, n= 2, R₂ = methoxy, R₃ = R₄ = H,

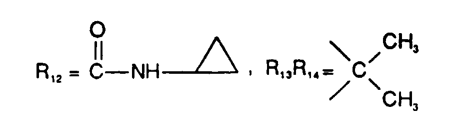

### Exemple 48 : β-D-Ribofuranuronamide, 1-[6-[[2-[1-cyclopentyl indol-3-yl] ethyl] amino]-9H-purin-9-yl]-N-cyclopropyl-1-deoxy-2,3-O-(1-methylethylidene)

**Formule (IV) :** R₁ = H, n= 0, R₂ = cyclopentyl, R₃ = R₄ = H,

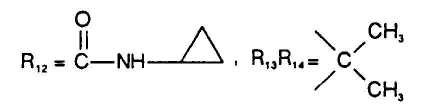

### Exemple 49 : β-D-Ribofuranuronamide, N-cyclopropyl-1-deoxy-1-[6-[[2-[1-isopropyl indol-3-yl] ethyl] amino]-9H-purin-9-yl]-2,3-O-(1-methylethylidene)

**Formule (IV) :** R₁ = H, n= 0, R₂ = isopropyl, R₃ = R₄ = H,

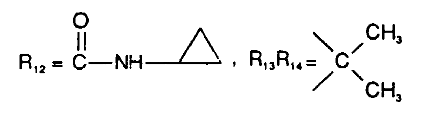

### Exemple 50 : β-D-Ribofuranuronamide, N-cyclopropyl-1-deoxy-1-[6-[[2-[1-(4-methylbenzyl) indol-3-yl] ethyl] amino]-9H-purin-9-yl] -2,3-O-(1-methylethylidene)

**Formule (IV) :** R₁ = H, n= 1, R₂ = 4-methyl phenyl, R₃ = R₄ = H,

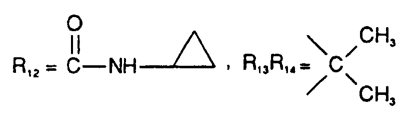

### Exemple 51 : β-D-Ribofuranuronamide, N-cyclopropyl-1-deoxy-1-[6-[[2-[1-(3,4-dimethyl benzyl) indol-3-yl] ethyl] amino]-9H-purin-9-yl]-2,3-O-(1-methylethylidene)

**Formule (IV) :** R₁ = H, n= 1, R₂ = 3,4-dimethylphenyl, R₃ = R₄ = H,

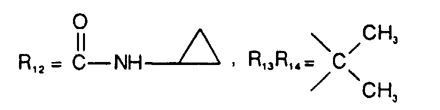

### Exemple 52 : β-D-Ribofuranuronamide, N-cyclopropyl-1-deoxy-1-[6-[[2-[1-(2,5-dimethylbenzyl) indol-3-yl] ethyl] amino]-9H-purin-9-yl]-2,3-O-(1-methylethylidene)

**Formule (IV) :** R₁ = H, n= 1, R₂ = 2,5-dimethylphenyl, R₃ = R₄ = H,

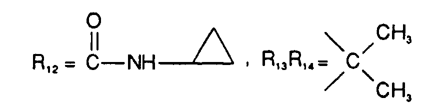

### Exemple 53 : β-D-Ribofuranuronamide, N-cyclopropyl-1-deoxy-1-[6-[[2-[1-(2-N-morphollnoethyl) indol-3-yl] ethyl] amino]-9H-purin-9-yl]-2,3-O-(1-methylethylidene)

**Formule (IV) :** R₁ = H, n= 2, R₂ = morpholino, R₃ = R₄ = H,

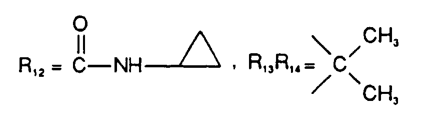

### Exemple 54 : β-D-Ribofuranuronamide, N-cyclopropyl-1-deoxy-1-[6-[[2-[1-(2-N,N-dimethyl amino ethyl) indol-3-yl] ethyl] amino]-9H -purin-9-yl]-2,3-O-(1-methylethylidene)

**Formule (IV) :** R₁ = H, n= 2, R₂ = N,N dimethylamino, R₃ = R₄ = H,

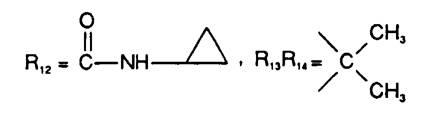

### Exemple 55 : β-D-Ribofuranuronamide, N-cyclopropyl-1-deoxy-2,3-O-(1-methylethylidene)1-[6-[[2-[1-(2- N-piperidinoethyl) indol-3-yl] ethyl] amino]-9H-purin-9-yl]

**Formule (IV) :** R₁ = H, n= 2, R₂ = N-piperidino, R₃ = R₄ = H,

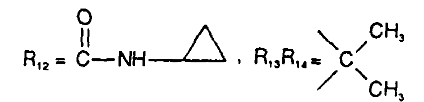

### Exemple 56 : β-D-Ribofuranuronamide, N-cyclopropyl-1-deoxy-2,3-O-(1-methylethylidene)1-[6-[[2-[1-(2-N-pyrrolidinoethyl) indol-3-yl] ethyl] amino]-9H-purin-9-yl]

**Formule (IV) :** R₁ = H, n= 2, R₂ = N-pyrrolidino, R₃ = R₄ = H,

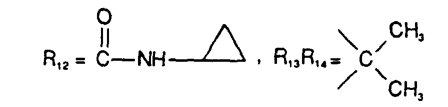

### Exemple 57 : β-D-Ribofuranuronamide, N-cyclopropyl-1-deoxy-1-[6-[[2-[1-(3,4-dichloro benzyl) indol-3-yl] ethyl] amino]-9H-purin-9-yl]-2,3-O-(1-methylethylidene)

**Formule (IV) :** R₁ = H, n= 1, R₂ = 3,4-dichlorophenyl, R₃ = R₄ = H,

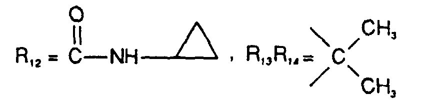

### Exemple 58 : β-D-Ribofuranuronamide, N-cyclopropyl-1-deoxy-2,3-O-(1-methylethylidene) 1-[6-[[2-[1-(3-pyridyl methyl) indol-3-yl] ethyl] amino]-9H- purin-9-yl]

**Formule (IV) :** R₁ = H, n= 1, R₂ = 3-pyridyl, R₃ = R₄ = H,

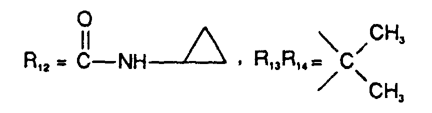

### Exemple 59 : β-D-Ribofuranuronamide, N-cyclopropyl-1-deoxy-2,3-O-(1-methylethylldene) 1-[6-[[2-[1-(1-naphtylmethyl) indol-3 -yl] ethyl] amino]-9H-purin-9-yl]

**Formule (IV) :** R₁ = H, n= 1, R₂ = 1-naphtyl, R₃ = R₄ = H,

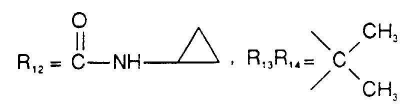

Selon le mode opératoire de l'exemple 46 mais en utilisant l'uronamide préparé à l'exemple 3, le composé de l'exemple 60 a été préparé.

### Exemple 60 : β-D-Ribofuranuronamide, 1-[6-[[2-[1-(4-chlorobenzyl) indol-3-yl] ethyl] amino]-9H-purin-9-yl]-1-deoxy-N-(1,1-dimethyl 2-hydroxy ethyl)-2,3-O-(1-methylethylidene)

**Formule (IV) :** R₁ = H, n= 1, R₂ = 4-chlorophenyl, R₃ = R₄ = H,

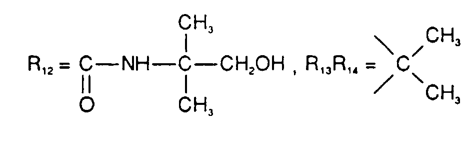

### Exemple 61 : β-D-Ribofuranuronamide, 1-[6-[[2-[1-(4-chlorobenzyl) indol-3-yl] ethyl] amino]-9H-purin-9-yl]-N-cyclopropyl-1-deoxy

**Formule (I) :** R₁ = H, n= 1, R₂ = 4-chlorophenyl, R₃ = R₄ = H,

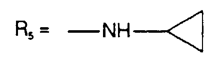

Placer 7.2 g de purine obtenus à l'exemple 46 dans 135 ml de HCl 1N. Chauffer à 60°C pendant 3 h. Refroidir. Décanter la solution pour séparer la phase aqueuse de la gomme plus ou moins visqueuse formée. Neutraliser la phase aqueuse avec une solution de bicarbonate de sodium. Extraire au chloroforme. Regrouper les phases organiques avec la gomme précédemment obtenue. Laver à l'eau, sécher, concentrer pour obtenir 7 g d'un solide crème.

Le composé est purifié par chromatographie sur gel de silice (éluant chloroforme 95 % / méthanol 5 %) pour donner 3.7 g de β-D-Ribofuranuronamide, 1-[6-[[2-[1-(4-chlorobenzyl) indol-3-yl] ethyl] amino]-9H-purin-9-yl]-N-cyclopropyl-1-deoxy.
Formule brute : C₃₀H₃₀Cl N₇O₄
Point de fusion : 225°C.

Le même composé 61 peut être obtenu par hydrolyse en milieu acide formique (212 ml d'une solution à 50 %) en chauffant à 70°C pendant 75 mn.

Selon l'exemple 61, les composés des exemples 62 à 75 ont été préparés.

### Exemple 62 : β-D-Ribofuranuronamide, 1-[6-[[2-[1-(4-chlorobenzyl) indol-3-yl] ethyl] amino]-9H-purin-9-yl]-1-deoxy-N-(1,1 dimethyl 2-hydroxy ethyl)

**Formule (I) :** R₁ = H, n= 1, R₂ = 4-chlorophenyl, R₃ = R₄ = H.

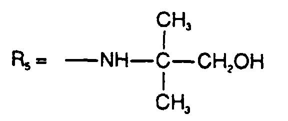

Purifié par deux chromatographies successives sur gel de silice (éluant chloroforme 90 % / methanol 10 %).
Formule brute : C₃₁H₃₄ Cl N₇O₅
Point de fusion : 189°C.

### Exemple 63 : β-D-Ribofuranuronamide, N-cyclopropyl-1-deoxy-1-[6-[[2-[1-(2-methoxyethyl) indol-3-yl] ethyl] amino]-9H-purin-9-yl]

**Formule (I) :** R₁ = H, n= 2, R₂ = methoxy, R₃ = R₄ = H,

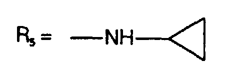

Purifié par chromatographie sur gel de silice (éluant chloroforme 95 %/méthanol 5 %).
Formule brute C₂₆H₃₁N₇O₅.
Point de fusion : 132°C.

### Exemple 64 : β-D-Ribofuranuronamide, 1-[6-[[2-[1-cyclopentyl indol-3-yl] ethyl] amino]-9H-purin-9-yl]-N-cyclopropyl-1-deoxy

**Formule (I) :** R₁ = H, n= 0, R₂ = cyclopentyl, R₃ = R₄ = H,

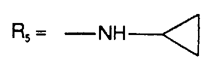

Purifié par chromatographie sur gel de silice (éluant chloroforme 95 % / méthanol 5 %).
Formule brute C₂₈H₃₃N₇O₄.
Point de fusion : 141°C.

### Exemple 65 : β-D-Ribofuranuronamide, N-cyclopropyl-1-deoxy-1-[6-[[2-[1-isopropyl indol-3-yl] ethyl] amino]-9H-purin-9-yl]

**Formule (I) :** R₁ = H, n= 0, R₂ = isopropyl, R₃ = R₄ = H,

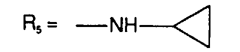

Purifié par chromatographie sur gel de silice (éluant chloroforme 90 % / méthanol 10 %).
Formule brute C₂₆H₃₁N₇O₄.
Point de fusion: 135°C.

### Exemple 66 : β-D-Ribofuranuronamide, N-cyclopropyl-1-deoxy-1-[6-[[2-[1-(4-methylbenzyl) indol-3-yl] ethyl] amino]-9H-purin-9-yl]

**Formule (I) :** R₁ = H, n= 1, R₂ = 4-methyl phenyl, R₃ = R₄ = H,

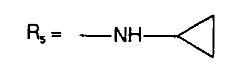

Purifié par chromatographie sur gel de silice (éluant chloroforme 90 % / méthanol 10 %).
Formule brute : C₃₁H₃₃N₇O₄,H₂O
Point de fusion : 144°C.

### Exemple 67 : β-D-Ribofuranuronamide, N-cyclopropyl-1-deoxy-1-[6-[[2-[1-(3,4-dimethyl benzyl) indol-3-yl] ethyl] amino]-9H-purin-9-yl]

**Formule (I) :** R₁ = H, n= 1, R₂ = 3,4-dimethylphenyl, R₃ = R₄ = H,

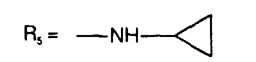

Purifié par chromatographie sur gel de silice (éluant chloroforme 95 % / méthanol 5 %).
Formule brute : C₃₂H₃₅N₇O₄, H₂O
Point de fusion : 134°C.

### Exemple 68 : β-D-Ribofuranuronamide, N-cyclopropyl-1-deoxy-1-[6-[[2-[1-(2,5-dimethyl benzyl) indol-3- yl] ethyl] amino]-9H-purin-9-yl]

**Formule (I) :** R₁ = H, n= 1, R₂ = 2,5-dimethylphenyl, R₃ = R₄ = H,

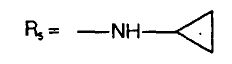

Purifié par chromatographie sur gel de silice (éluant chloroforme 95 % / méthanol 5 %).
Formule brute : C₃₂H₃₅N₇O₄, 0.5 H₂O
Point de fusion : 130°C.

### Exemple 69 : β-D-Ribofuranuronamide, N-cyclopropyl-1-deoxy-1-[6-[[2-[1-(2-N-morpholinoethyl) indol-3- yl] ethyl] amino]-9H-purin-9-yl]

**Formule (I)** : R₁ = H, n= 2, R₂ = N-morpholino, R₃ = R₄ = H,

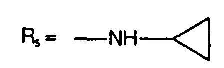

Purifié par chromatographie sur gel de silice (éluant chloroforme 95 % / méthanol 10 %).
Formule brute : C₂₉H₃₆N₈O₅, 0.5 H₂O
Point de fusion : 109-110°C

### Exemple 70 : β-D-Ribofuranuronamide, N-cyclopropyl-1-deoxy-1-[6-[[2-[1-(2-N,N-dimethyl amino ethyl) indol-3-yl] ethyl] amino]-9H-purin-9-yl]

**Formule (I)** : R₁ = H, n= 2, R₂ = N,N dimethylamino, R₃ = R₄ = H,

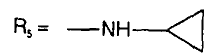

Purifié par chromatographie sur gel de silice (éluant chloroforme 80 % / isopropylamine 20 %
Formule brute : C₂₇H₃₄N₈O₄, 0.5 H₂O
Point de fusion : 112°C.

### Exemple 71 : β-D-Ribofuranuronamide, N-cyclopropyl-1-deoxy-1-[6-[[2-[1-(2-N-piperidinoethyl) indol-3-yl] ethyl] amino]-9H-purin-9-yl]

**Formule (I) :** R₁ = H, n= 2, R₂ = N-piperidino, R₃ = R₄ = H,

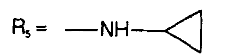

Purifié par chromatographie sur gel de silice (éluant chloroforme 80 % / methanol 20 %).
Formule brute : C₃₀H₃₈ N₈O₄
Point de fusion : 109°C.

A une solution de 3,7 g d'acide citrique dans 40 ml d'éthanol, on introduit goutte à goutte 11 g du composé ainsi préparé en solution dans 100 ml d'éthanol. Agiter 1 h à température ambiante. Essorer le solide formé, laver à l'éthanol, sécher pour obtenir 10,6 g de citrate de β-D-Ribofuranuronamide, N-cyclopropyl-1-deoxy-1-[6-[[2-[1-(2-N-piperidino ethyl) indol-3-yl] ethyl] amino]-9H-purin-9-yl].
Formule brute : C₃₀H₃₈N₈O₄, C₆H₈O₇
Point de fusion : 138°C

### Exemple 72 : β-D-Ribofuranuronamide, N-cyclopropyl-1-deoxy-1-[6-[[2-[1-(2-N-pyrrolidinoethyl) indol-3-yl] ethyl] amino]-9H-purin-9-yl]

**Formule (I) :** R₁ = H, n= 2, R₂ = N-pyrrolidino, R₃ = R₄ = H,

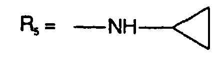

Purifié par chromatographie sur gel de silice (éluant chloroforme 80 % / methanol 20 %).
Formule brute C₂₉H₃₆N₈O₄ ; 0.5 H₂O
Point de fusion : 126°C.

### Exemple 73 : β-D-Ribofuranuronamide, N-cyclopropyl-1-deoxy 1-[6-[[2-[1-(3,4-dichlorobenzyl) indol-3-yl] ethyl] amino]-9H-purin-9-yl]

**Formule (I) :** R₁ = H, n= 1, R₂ = 3,4-dichlorophenyl, R₃ = R₄ = H,

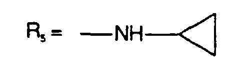

Purifié par chromatographie sur gel de silice (éluant chloroforme 95 % / méthanol 5 %).
Formule brute : C₃₀H₂₉Cl₂N₇O₄, 0.8 H₂O
Point de fusion : 141°C.

### Exemple 74 : β-D-Ribofuranuronamide, N-cyclopropyl-1-deoxy-1-[6-[[2-[1-(3-pyridylmethyl) indol-3- yl] ethyl] amino]-9H-purin-9-yl]

**Formule (I)** : R₁ = H, n= 1, R₂ = 3-pyridyl, R₃ = R₄ = H,

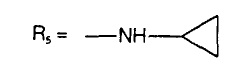

Purifié par recristallisation dans le 2-methoxy ethanol.
Formule brute : C₂₉H₃₀N₈O₄ , 0.5 CH₃O CH₂CH₂OH
Point de fusion : 239°C.

### Exemple 75 : β-D-Ribofuranuronamide, N-cyclopropyl-1-deoxy-1-[6-[[2-[1-(1-naphtylmethyl) indol-3- yl] ethyl] amino]-9H-purin-9-yl]

**Formule (I) :** R₁ = H, n= 1, R₂ = 1-naphtyl, R₃ = R₄ = H,

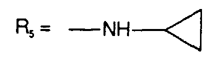

Purifié par chromatographie sur gel de silice (éluant : chloroforme 95 % / méthanol 5 %) puis recristallisation dans l'isopropanol.
Formule brute : C₃₄ H₃₃N₇O₄
Point de fusion : 168°C.

### Exemple 76 : N⁶-[2-[1-(4-chlorobenzyl) indol-3-yl] ethyl] adenosine

**Formule (IV) :** R₁ = H, n = 1, R₂ = 4-chlorophenyl, R₃ = R₄ = H, R₁₂ = CH₂OH, R₁₃ = R₁₄ = H

On place dans 100 ml d'éthanol 4.5 g de chlorhydrate de 1-(4-chlorobenzyl) 3-(2-aminoethyl) indole préparés à l'exemple 29 ou 30. Rajouter 2.1 g de triéthylamine puis 2 g de 6-chloro adenosine.

Porter l'ensemble au reflux pendant 6 h. Refroidir. Le précipité obtenu est filtré, lavé à l'éthanol puis à l'éther.

Une recristallisation dans l'éthanol conduit à 2.5 g de N₆-[2-[1-(4-chlorobenzyl) indol-3-yl] ethyl] adenosine.

Point de fusion: 181°C.

Selon l'exemple 76, les composés des exemples 77 et 78 ont été préparés :

### Exemple 77 : N⁶-[2-[1-benzyl indol-3-yl] ethyl] adenosine

**Formule (IV)** : R₁ = H, n = 1, R₂ = phényl, R₃ = R₄ = H, R₁₂ = CH₂OH, R₁₃ = R₁₄ = H

Purifié par recristallisation dans l'éthanol.
Point de fusion : 158°C.

### Exemple 78 : N⁶-[2-[1-(2,6-dichlorobenzyl) indol-3-yl] ethyl] adenosine

**Formule (IV)** : R₁ = H, n = 1, R₂ = 2,6-dichlorophényl, R₃ = R₄ = H, R₁₂ = CH₂OH, R₁₃ = R₁₄ = H

Purifié par recristallisation dans l'éthanol.
Point de fusion : 192°C.

Les alcools des exemples 76, 77 et 78 pourront être oxydés en acide par action d'un agent oxydant tel que l'anhydride chromique dans l'acétone en présence d'acide sulfurique ou le permanganate de potassium dans l'eau en présence d'ammoniaque. Ils conduiront ensuite aux chlorures d'acides correspondants après réaction avec le chlorure de thionyle et puis par réaction avec des amines appropriées aux dérivés ribofuranuronamides du même type que ceux des exemples 61, 62, 73 ou 75.

Selon le mode opératoire de l'exemple 61, les composés des exemples 79 à 100 ont été préparés.

### Exemple 79 : β-D-Ribofurauronamide, N-cyclopropyl-1-deoxy-1 [6-[[2-[1-(2-pyridyl méthyl) indol-3-yl] ethyl] amino]-9H-purin-9-yl]

**Formule (I)** : R₁ = H ; n = 1 ; R₂ = 2-pyridyl, R₃ = R₄ = H ;

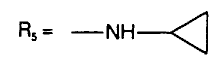

Purifié par trois colonnes successives (éluant chloroforme 90 % / méthanol 10 % ; chloroforme 80 % / isopropylamine 20 % et dichlorométhane 90 % / méthanol 10 % respectivement).
Formule brute : C₂₉H₃₀ N₈O₄
Point de fusion : 122°C

### Exemple 80 : β-D-Ribofuranuronamide, 1-[6-[[2-[1-(4-chlorobenzyl) 5-chloro indol-3-yl] ethyl] amino]-9H-purin-9-yl]-N-cyclopropyl-1-deoxy

**Formule (I)** : R₁ = 5-Cl; n = 1; R₂ = 4-chlorophényl ; R₃ = R₄ = H ;

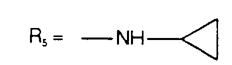

Purifié par cristallisation dans un mélange d'isopropanol/éther.
Formule brute : C₃₀H₂₉Cl₂N₇O₄
Point de fusion : 154°C

### Exemple 81 : β-D-Ribofuranuronamide, 1-[6-[[2-[1-(2,5-diméthyl benzyl) 5-chloro indol-3-yl] ethyl] amino]-9H-purin-9-yl]-N-cyclopropyl-1-deoxy

**Formule (I)** : R₁ = 5-Cl ; n = 1 ; R₂ = 2,5-diméthyl phényl, R₃ = R₄ = H ;

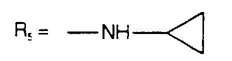

Recristallisation dans l'éthanol avec passage au noir animal.
Formule brute : C₃₂ H₃₄ Cl N₇O₄ ; 1.1 H₂O
Point de fusion : 139°C

### Exemple 82 : β-D-Riboturanuronamide, 1-[6-[[2-[1-(4-chlorobenzyl) indol-3-yl] ethyl] amino]-9H-purin-9-yl]-1-deoxy N-(2-méthoxy éthyl)

**Formule (I) :** R₁ = H ; n = 1 ; R₂ = 4-chlorophényl ; R₃ = R₄ = H ; R₅ = -NH-CH₂-CH₂-OCH₃

Purifié par traitement à chaud dans l'éthanol.
Formule brute : C₃₀ H₃₂Cl N₇O₅
Point de fusion : 193°C

### Exemple 83 : β-D-Ribofuranuronamide, N-cyclopropyl-1-deoxy-1-[6-[[2-[1-allyl indol-3-yl] ethyl] amino]-9H-purin-9-yl]

**Formule (I) :** R₁ = H ; n =1 ; R₂ = -HC=CH₂ ; R₃ = R₄ = H;

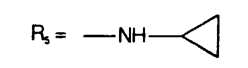

Purifié par chromatographie sur gel de silice (éluant chloroforme 90 % / méthanol 10 %).
Formule brute : C₂₆H₂₉N₇O₄ 0.9 H₂O
Point de fusion : 117°C

### Exemple 84 : β-D-Ribofuranuronamide, N-cyclopropyl-1-deoxy-1-[6-[[2-[1-(2-propynyl) indol-3-yl] ethyl] amino]-9H-purin-9-yl]

**Formule (I) :** R₁ = H ; n = 1 ; R₂ = -C≡CH ; R₃ = R₄ = H ;

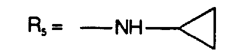

Purifié par chromatographie sur gel de silice (éluant chloroforme 90 % / méthanol 10 %).
Formule brute : C₂₆H₂₇N₇O₄ ; H₂O
Point de fusion : 123°C

### Exemple 85 : β-D-Ribofuranuronamide, N-cyclopropyl-1-deoxy-1-[6-[[2-[1-(2,5-diméthylbenzyl) 5-méthyl indol-3-yl] ethyl] amino]-9H-purin-9-yl]

**Formule (I) :** R₁ = 5-CH₃ ; n = 1; R₂ = 2,5-diméthyl phényl ; R₃ = R₄ = H ;

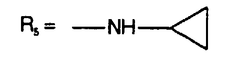

Purifié par chromatographie sur gel de silice (éluant chloroforme 90 % / méthanol 10 %).
Formule brute : C₃₃H₃₇N₇O₄ ;0.8 H₂O
Point de fusion : 129°C

### Exemple 86 : β-D-Ribofuranuronamide, N-cyclopropyl-1-deoxy-1-[6-[[2-[1-(2,5-diméthylbenzyl) 5-méthoxy indol-3-yl] ethyl] amino]-9H-purin-9-yl]

**Formule (I) :** R₁ = 5-OCH₃ ; n = 1 ; R₂ = 2,5-diméthyl phényl ; R₃ = R₄ = H;

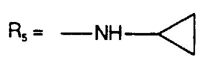

Purifié par chromatographie sur gel de silice (éluant chloroforme 90 % / méthanol 10 %).
Formule brute : C₃₃H₃₇N₇O₅; 0.1 H₂O
Point de fusion : 182°C

### Exemple 87 : β-D-Ribofuranuronamide, N-cyclopropyl-1-deoxy-1-[6-[[2-[1-(2,5-diméthylbenzyl)-2-méthyl indol-3-yl] ethyl] amino]-9H-purin-9-yl]

**Formule (I)** : R₁ = 2-CH₃ ; n = 1 ; R₂ = 2,5-diméthyl phényl ; R₃ = R₄ = H;

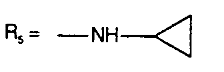

Purifié par chromatographie sur gel de silice (éluant chloroforme 90 % / méthanol 10 %).
Formule brute : C₃₃H₃₇N₇O₄; 0.7 H₂O
Point de fusion : 144°C

### Exemple 88 : β-D-Ribofuranuronamide, N-cyclopropyl-1-deoxy-1-[6-[[2-[1-(4-méthoxy benzyl) indol-3-yl] ethyl] amino]-9H-purin-9-yl]

**Formule (I)** : R₁ = H ; n = 1 ; R₂ = 4-OCH₃ phényl ; R₃ = R₄ = H;

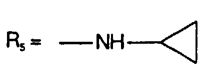

Purifié par chromatographie sur gel de silice (éluant chloroforme 90 % / méthanol 10 %).
Formule brute : C₃₁H₃₃N₇O₅; 0.8 H₂O
Point de fusion : 134°C

### Exemple 89 : β-D-Ribofuranuronamide, 1-[6-[[2-[1-cyclopentyl-2-méthyl indol-3-yl] ethyl] amino]-9H-purin-9-yl]-N-cyclopropyl-1-deoxy

**Formule (I) :** R₁ = 2-CH₃ ; n = 0 ; R₂ = cyclopentyl ; R₃ = R₄ = H;

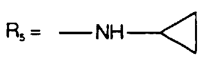

Purifié par chromatographie sur gel de silice (éluant chloroforme 90 % / méthanol 10 %).
Formule brute : C₂₉H₃₅N₇O₄
Point de fusion : 140°C

### Exemple 90 : β-D-Ribofuranuronamide, N-cyclopropyl-1-deoxy-1-[6-[[2-[1-(2-N,N-diméthyl amino benzyl) indol-3-yl ethyl] amino]-9H-purin-9-yl]

**Formule (I)** : R₁ = H ; n = 1 ; R₂ = 2-N,N-diméthylamino phényl ; R₃ = R₄ = H;

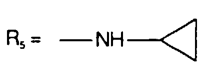

Purifié par chromatographie sur gel de silice (éluant chloroforme 90 % / méthanol 10 %)
Formule brute : C₃₂H₃₆N₈O₄
Point de fusion : 128-129°C

### Exemple 91 : β-D-Ribofuranuronamide, N-cyclopropyl-1-deoxy-1-[6-[[2-[1-(3-nitrobenzyl) indol-3-yl ethyl] amino]-9H-purin-9-yl]

**Formule (I) :** R₁ = H ; n = 1 ; R₂ = 3-NO₂ phényl ; R₃ = R₄ = H ;

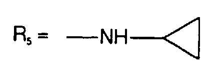

Purifié par deux chromatographies successives (éluant chloroforme 90 % / méthanol 10 % et dichlorométhane 90 % / méthanol 10 % respectivement).
Formule brute : C₃₀H₃₀N₈O₆ ; 0.3 H₂O
Point de fusion : 129°C

### Exemple 92 : β-D-Ribofuranuronamide, N-cyclopropyl-1-deoxy-1-[6-[[1-[1-(2,5-diméthyl benzyl) indol-3-yl] propan-2-yl] amino-9H-purin-9-yl]

**Formule (I)** : R₁ = H ; n = 1 ; R₂ = 2,5-diméthyl phényl ; R₃ = H ; R₄ = CH₃ ;

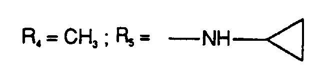

Purifié par chromatographie sur gel de silice (éluant chloroforme 90 % / méthanol 10 %).
Formule brute : C₃₃H₃₇N₇O₄
Point de fusion : 135°C

### Exemple 93 : β-D-Ribofuranuronamide, N-cyclopropyl-1-deoxy-1-[6-[[1-[1-cyclopentyl indol-3-yl] propan-2-yl] amino]-9H-purin-9-yl]

**Formule (I)** : R₁ = H ; n = 0 ; R₂ = cyclopentyl; R₃ = H ; R₄ = CH₃ ;

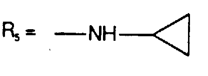

Purifié par chromatographie sur gel de silice (éluant chloroforme 90 % / méthanol 10 %).
Formule brute : C₂₉H₃₅N₇O₄
Point de fusion : 130°C

### Exemple 94 : β-D-Ribofuranuronamide, N-cyclopropyl-1-deoxy-1-[6-[[2-[1-(2,5-diméthyl benzyl) indol-3-yl] propyl] amino]-9H-purin-9-yl]

**Formule (I)** : R₁ = H ; n =1 ; R₂ = 2,5-diméthylphényl ; R₃ = CH₃ ; R₄ = H ;

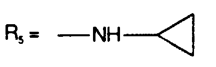

Purifié par chromatographie sur gel de silice (éluant chloroforme 95 % / méthanol 5 %)
Formule brute : C₃₃H₃₇N₇O₄
Point de fusion : 137°C

### Exemple 95 : β-D-Ribofuranuronamide, N-cyclopropyl-1-deoxy-1-[6-[[2-[1-(2,5-diméthyl benzyl)-2-phényl indol-3-yl] éthyl] amino]-9H-purin-9-yl]

**Formule (I) :** R₁ = 2-phényl ; n = 1 ; R₂ = 2,5-diméthylphényl ; R₃ = R₄ = H ;

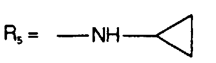

Purifié par chromatographie sur gel de silice (éluant chloroforme 90 %/ méthanol 10 %).
Formule brute : C₃₈H₃₉N₇O₄
Point de fusion : 136°C

### Exemple 96 : β-D-Ribofuranuronamide, N-cyclopropyl-1-deoxy-1-[6-[[2-[1-(2,5-diméthyl benzyl)-5-thiométhyl indol-3-yl] éthyl] amino]-9H-purin-9-yl]

**Formule (I) :** R₁ = 5-SCH₃ ; n =1 ; R₂ = 2,5-diméthylphényl ; R₃ = R₄ = H ; 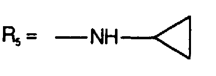 Formule brute : C₃₃H₃₇N₇O₄S, 0.8 H₂O
Point de fusion : 137°C

### Exemple 97 : β-D-Ribofuranuronamide,1-[6-[[2-[1-(5-chloro-2-thiényl) indol-3-yl] éthyl] amino]-9H-purin-9-yl] N-cyclopropyl-1-deoxy

**Formule (I)** : R₁ = H ; n = 1; R₂ = 5-chloro-2-thiényl ; R₃ = R₄ = H ;

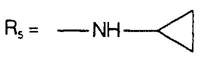

Purifié par chromatographie sur gel de silice (éluant chloroforme 90 % / méthanol 10 %)
Formule brute : C₂₈H₂₈ Cl N₇O₄S, 1 H₂O
Point de fusion : 137°C

### Exemple 98 : β-D-Ribofuranuronamide, N-cyclopropyl-1-[6-[[2-[1-(cyclo propyl méthyl) indol-3-yl] éthyl] amino]-9H-purin-9-yl]-1-deoxy

**Formule (I)** : R₁ = H ; n = 1 ; R₂ = cyclopropyl ; R₃ = R₄ = H ; 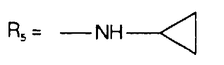 Formule brute : C₂₇H₃₁N₇O₄, 0.5 H₂O
Point de fusion : 134°C

Selon le mode opératoire de l'exemple 46 et en utilisant l'uronamide de formule (III) approprié, les dérivés des exemples 99 à 118 ont été préparés sous forme de solides amorphes.

### Exemple 99 : β-D-Ribofuranuronamide, N-cyclopropyl-1-deoxy-1-[6-[[2-[1-(2-pyridyl méthyl) indol-3-yl] éthyl] amino]-9H-purin-9-yl] 2,3-O-(1-méthyléthylidène)

**Formule (IV) :** R₁ = H ; n = 1; R₂ = 2-pyridyl ; R₃ = R₄ = H ;

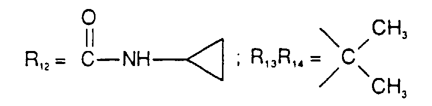

### Exemple 100 : β-D-Ribofuranuronamide, 1-[6-[[2-[1-(4-chlorobenzyl) 5- chloro indol-3-yl] éthyl] amino]-9H-purin-9-yl]-N-cyclopropyl-1-deoxy-2,3-O-(1-méthyléthylidène)

**Formule (IV) :** R₁ = 5-Cl ; n = 1 ; R₂ = 4-chlorophényl ; R₃ = R₄ = H ;

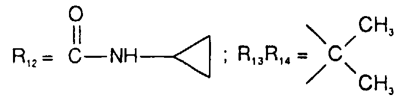

### Exemple 101 : β-D-Ribofuranuronamide,1-[6-[[2-[1-(2,5-diméthyl benzyl) 5- chloro indol-3-yl] éthyl] amino]-9H-purin-9-yl]-N-cyclopropyl-1-deoxy-2,3-O-(1-méthyléthylidène)

**Formule (IV)** : R₁ = 5-Cl ; n = 1; R₂ = 2,5-diméthylphényl ; R₃ = R₄ = H ;

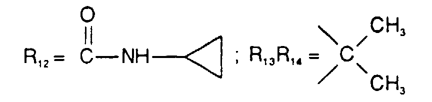

### Exemple 102 : β-D-Ribofuranuronamide,1-[6-[[2-[1-(4-chlorobenzyl) indol-3-y] éthyl] amino]-9H-purin-9-yl]-1-deoxy N-(2-méthoxy éthyl)-2,3-O-(1-méthyléthylidène)

**Formule (IV) :** R₁ = H ; n = 1 ; R₂ = 4-chlorophényl ; R₃ = R₄ = H ;

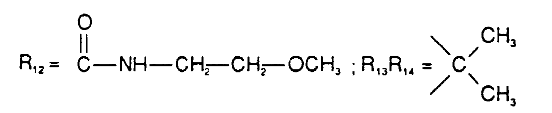

### Exemple 103 : β-D-Ribofuranuronamide, N-cyclopropyl-1-deoxy-1-[6-[[2-[1-allyl indol-3-yl]éthyl] amino]-9H-purin-9-yl]-2,3-O-(1-méthyléthylidène)

**Formule (IV)** : R₁ = H ; n = 1 ; R₂ = éthényl ; R₃ = R₄ = H ;

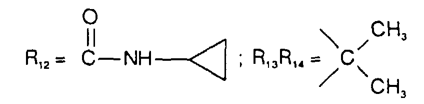

### Exemple 104 : β-D-Ribofuranuronamide, N-cyclopropyl-1-deoxy-1-[6-[[2-[1-propargyl indol-3-yl]éthyl] amino]-9H-purin-9-yl]-2,3-O-(1-méthyléthylidène)

**Formule (IV)** : R₁ = H ; n = 1 ; R₂ = éthynyl; R₃ = R₄ = H;

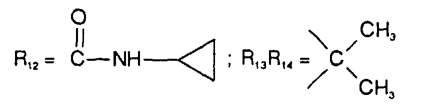

### Exemple 105 : β-D-Ribofuranuronamide, N-cyclopropyl-1-deoxy-1-[6-[[2-[2,5-diméthylbenzyl) 5-méthyl indol-3-yl]éthyl] amino]-9H-purin-9-yl]-2,3-O-(1-méthyléthylidène)

**Formule (IV)** : R₁ = 5-CH₃ ; n = 1 ; R₂ = 2,5-diméthylphényl ; R₃=R₄ =H ;

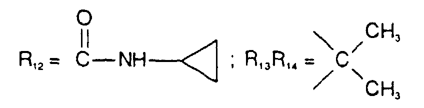

### Exemple 106 : β-D-Ribofuranuronamide, N-cyclopropyl-1-deoxy-1-[6-[[2-[1-(2,5-diméthylbenzyl) 5-méthoxy indol-3-yl]éthyl] amino]-9H-purin-9-yl]-2,3-O-(1-méthyléthylidène)

**Formule (IV)** : R₁ = 5-OCH₃ ; n = 1 ; R₂ = 2,5-diméthylphényl ; R₃ = R₄ = H ;

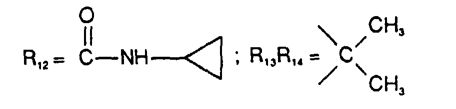

### Exemple 107 : β-D-Ribofuranuronamide, N-cyclopropyl-1-deoxy-1-[6-[[2-[1-(2,5-diméthylbenzyl)-2-méthyl indol-3-yl]éthyl] amino]-9H-purin-9-yl]-2,3-O-(1-méthyléthylidène)

**Formule (IV)** : R₁ = 2-CH₃ ; n =1 ; R₂ = 2,5-diméthylphényl ; R₃ = R₄ = H ;

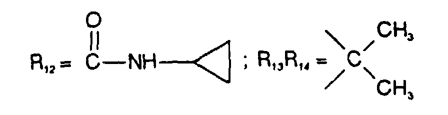

### Exemple 108 : β-D-Ribofuranuronamide, N-cyclopropyl-1-deoxy-1-[6-[[2-[1-(4-méthoxybenzyl) indol-3-yl]éthyl] amine]-9H-purin-9-yl]-2,3-O-(1-méthyléthylidène)

**Formule (IV)** : R₁ = H ; n = 1 ; R₂ = 4-OCH₃phényl ; R₃ = R₄ = H ;

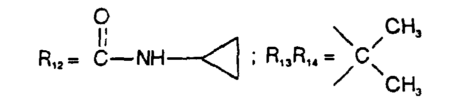

### Exemple 109 : β-D-Ribofuranuronamide,1-[6-[[2-[1-cyclopentyl-2-méthyl indol-3-yl]éthyl] amino]-9H-purin-9-yl]-N-cyclopropyl-1-deoxy-2,3,O-(1-méthyléthylidène)

**Formule (IV)** : R₁ = 2-CH₃ ; n = 0 ; R₂ = cyclopentyl ; R₃ = R₄ = H ;

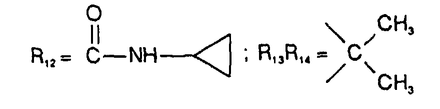

### Exemple 110 : β-D-Ribofuranuronamide, N-cyclopropyl-1-deoxy-1- [6-[[2-[1-(2-N,N-diméthyl amino benzyl) indol-3-yl]éthyl] amino]-9H-purin-9-yl]-2,3,O-(1- méthyléthylidène)

**Formule (IV) :** R₁ = H ; n = 1 ; R₂ = 2-N,N diméthylaminophényl ; R₃ = R₄ = H ;

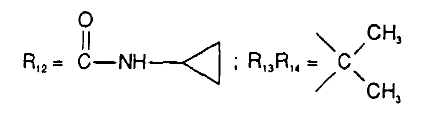

### Exemple 111 : β-D-Ribofuranuronamide, N-cyclopropyl-1deoxy-1- [6-[[2-[1-(3-nitrobenzyl) indol-3-yl]éthyl] amino]-9H-purin-9-yl]-2,3,O-(1-méthyléthylidène)

**Formule (IV) :** R₁ = H ; n = 1 ; R₂ = 3-nitrophényl ; R₃ = R₄ = H ;

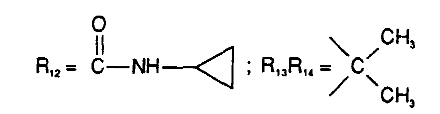

### Exemple 112 : β-D-Ribofuranuronamide, N-cyclopropyl-1-deoxy-1- [6-[[1-[1-(2,5-diméthyl benzyl) indol-3-yl]éthyl] propan-2-yl] amino]-9H-purin-9-yl]-2,3,O-(1- méthyléthylidène)

**Formule (IV) :** R₁ = H ; n = 1 ; R₂ = 2,5 diméthyl phényl ; R₃ = H ; R₄ =CH₃ ;

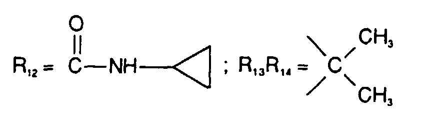

### Exemple 113 : β-D-Ribofuranuronamide, N-cyclopropyl-1-deoxy-1- [6-[[1-[1-cyclopentyl indol-3-yl] propan-2-yl] amino]-9H-purin-9-yl]-2,3,O-(1-méthyléthylidène)

**Formule (IV) :** R₁ = H ; n = 0 ; R₂ = cyclopentyl ; R₃ = H ; R₄ = CH₃ ;

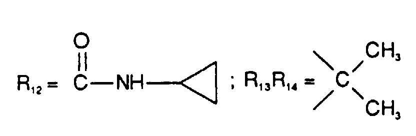

### Exemple 114 : β-D-Ribofuranuronamide, N-cyclopropyl-1-deoxy-1- [6-[[2-[1-(2,5-diméthylbenzyl) indol-3-yl] propyl] amino]-9H-purin-9-yl]-2,3,O-(1-méthyléthylidène)

**Formule (IV) :** R₁ = H ; n = R₂ = 2,5-diméthylphényl ; R₃=CH₃ ; R₄=H ;

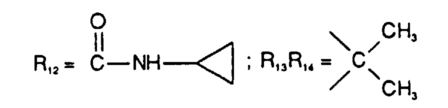

### Exemple 115 : β-D-Ribofuranuronamide, N-cyclopropyl-1-deoxy-1- [6-[[2-[1-(2,5-diméthylbenzyl)-2-phényl indol-3-yl] ethyl] amino]-9H-purin-9-yl]-2,3,O-(1- méthyléthylidène)

**Formule (IV) :** R₁ = 2-phényl ; n = 1 ; R₂ = 2,5-diméthylphényl ; R₃ = R₄ = H ;

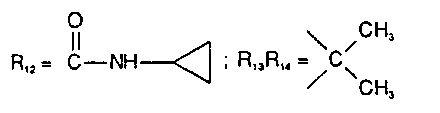

### Exemple 116 : β-D-Ribofuranuronamide, N-cyclopropyl-1-deoxy-1- [6-[[2-[1-(2,5-diméthylbenzyl)-5-thiométhyl indol-3-yl] ethyl] amino]-9H-purin-9-yl]-2,3,O-(1- méthyl éthylidène)

**Formule (IV) :** R₁ = 5-SCH₃ ; n = 1 ; R₂ = 2,5-diméthylphényl ; R₃ = R₄ = H;

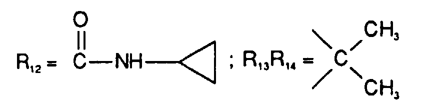

### Exemple 117 : β-D-Ribofuranuronamide, 1-[6-[[2-[1-(5-chlore-2-thiényl) indol-3-yl] éthyl] amino]-9H-purin-9-yl] N-cyclopropyl-1-deoxy-2,3,O-(1-méthyléthylidène)

**Formule (IV) :** R₁ = H ; n = 1 ; R₂ = 5-chloro-2-thiényl ; R₃ = R₄ = H ;

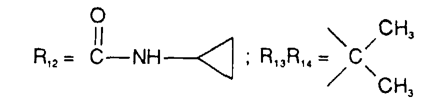

### Exemple 118 : β-D-Ribofuranuronamide, N-cyclopropyl-1-[6-[[2-[1-(cyclo propyl méthyl) indol-3-yl] éthyl] amino]-9H-purin-9-yl]-1-deoxy-2,3,O-(1-méthyléthylidène)

**Formule (IV) :** R₁ = H ; n = 1 ; R₂ = cyclopropyl ; R₃ = R₄ = H ; 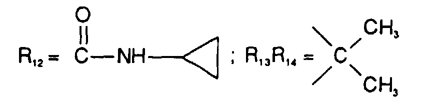 Selon l'un des modes opératoires des exemples 29 ou 30 les composés suivants des exemples 119 à 138 ont été préparés.

### Exemple 119 : 3-(2-aminoéthyl)-5-chloro-(2,5-diméthylbenzyl) indole

**Formule (II) :** R₁ = 5-Cl ; n = 1; R₂ = 2,5 diméthylphényl ; R₃ = R₄ = H

Huile brune purifiée par chromatographie sur gel de silice (éluant chloroforme 95 % / isopropylamine 5 %).

### Exemple 120 : 3-(2-aminoéthyl)-1-allyl indole

**Formule (II) :** R₁ = H ; n = 1; R₂ = éthényl ; R₃ = R₄ = H

Huile brune purifiée par chromatographie sur gel de silice (éluant chloroforme 95 % / isopropylamine 5 %).

### Exemple 121 : 3-(2-aminoéthyl)-1-(2-pyridyl méthyl) indole

**Formule (II) :** R₁ = H ; n = 1 ; R₂ = 2-pyridyl ; R₃ = R₄ = H Purifié par chromatographie sur gel de silice (éluant CHCl₃ 95 %/ isopropylamine 5 %).
Point de fusion : 237°C.

### Exemple 122 : 3-(2-aminoéthyl) 5-chloro-1-(4-chlorobenzyl) indole

**Formule (II) :** R₁ = 5-Cl ; n = 1 ; R₂ = 4-chlorophényl ; R₃ = R₄ = H Chlorhydrate, purifié par recristallisation dans l'éthanol.
Point de fusion : 204°C.

### Exemple 123 :3-(2-aminoéthyl)-1-propargyl indole

**Formule (II) :** R₁ = H ; n = 1 ; R₂ = éthynyl ; R₃ = R₄ =H

Huile brune purifiée par chromatographie sur gel de silice (éluant chloroforme 95 % / isopropylamine 5 %).

### Exemple 124 : 3-(2-aminoéthyl)-1-(2,5-diméthylbenzyl)-5-méthyl indole

**Formule (II) :** R₁ = 5-CH₃ ; n = 1 ; R₂ = 2,5-diméthylphényl ; R₃ = R₄ = H Chlorhydrate, purifié par cristallisation dans l'éther.
Point de fusion : 198°C.

### Exemple 125 : 3-(2-aminoéthyl)-1-(2,5-diméthylbenzyl)-5-méthoxy indole

**Formule (II) :** R₁ = 5-OCH₃ ; n = 1 ; R₂ = 2,5-diméthylphényl ; R₃ = R₄ = H

Solide blanc amorphe purifié par chromatographie sur gel de silice (éluant chloroforme 95 % / méthanol 5 %).

### Exemple 126 : 3-(2-aminoéthyl)-1-(2,5-diméthylbenzyl)-2-méthyl indole

**Formule (II) :** R₁ = 2-CH₃ ; n = 1; R₂ = 2,5-diméthylphényl ; R₃ = R₄ = H

Chlorhydrate, purifié par cristallisation dans l'éther.

Point de fusion : 250°C.

### Exemple 127 : 3-(2-aminoéthyl)-1-(4-méthoxy benzyl) indole

**Formule (II) :** R₁ = H ; n = 1; R₂ = 4-OCH₃phényl ; R₃ = R₄ = H

Huile brune purifiée par chromatographie sur gel de silice (éluant chloroforme 95 % / isopropylamine 5 %).

### Exemple 128 : 3-(2-aminoéthyl)-1-cyclopentyl 2-méthyl indole

**Formule (II) :** R₁ = 2-CH₃; n = 0 ; R₂ = cyclopentyl ; R₃ = R₄ = H

Huile orangée brute utilisée telle quelle à l'étape suivante.

### Exemple 129 : 3-(2-aminoéthyl)-2-phényl indole

**Formule (V) :** R₁ = 2-phényl ; R₃ = R₄=H Chlorhydrate, purifié par cristallisation dans l'isopropanol.
Point de fusion : 266°C.

### Exemple 130 : 3-(2-aminoéthyl)-1-(2,5-diméthylbenzyl)-2-phényl indole

**Formule (II) :** R₁ = 2-phényl ; n = 1; R₂ = 2,5-diméthylphényl ; R₃ = R₄ = H

Huile brune purifiée par chromatographie sur gel de silice (éluant chloroforme 95 % / isopropylamine 5 %).

### Exemple 131 : 3-(2-aminoéthyl)-1-(2- N,N-diméthylaminobenzyl) indole

**Formule (II) :** R₁ = H ; n = 1 ; R₂ = 2-N,N-diméthylaminophényl ; R₃ = R₄ = H

Huile brune purifiée par chromatographie sur gel de silice (éluant chloroforme 95 % / isopropylamine 5 %).

### Exemple 132 : 3-(2-aminoéthyl)-1-(3-nitrobenzyl) indole

**Formule (II) :** R₁ = H ; n = 1 ; R₂ = 3-NO₂ phényl ; R₃ = R₄ = H

Huile brune purifiée par chromatographie sur gel de silice (éluant chloroforme 95 % / isopropylamine 5 %).

### Exemple 133 : 3-(2-aminopropyl)-1-(2,5-diméthylbenzyl) indole

**Formule (II) :** R₁ = H ; n = 1; R₂ =2,5-diméthylphényl ; R₃ = H ; R₄ = CH₃

Solide blanc cristallisé dans l'éther isopropylique.
Point de fusion : 87°C.

### Exemple 134 : 3-(2-aminopropyl)-1-cyclopentyl indole

**Formule (II) :** R₁ = H ; n = 0 ; R₂ = cyclopentyl ; R₃ = H ; R₄ = CH₃

Huile orangée brute, utilisée telle quelle à l'étape suivante.

### Exemple 135 : 3-(aminopropan-2-yl)-1-(2,5-diméthylbenzyl) indole

**Formule (II) :** R₁ = H ; n = 1 ; R₂ = 2,5-diméthylphényl ; R₃ = CH₃ ; R₄ = H

Huile purifiée par chromatographie sur gel de silice (éluant chloroforme 95 % / isopropylamine 5 %). Chlorhydrate cristallisé dans l'isopropanol.
Formule brute : C₂₀H₂₄N₂ ; HCl
Point de fusion : 178°C

### Exemple 136 : 3-(aminoéthyl)-1-[(5-chloro 2-thiényl) méthyl] indole

**Formule (II) :** R₁ = H ; n = 1; R₂ = 5-chloro 2-thiényle; R₃ = R₄ = H

Huile brune purifiée par chromatographie sur gel de silice (éluant chloroforme 95 % / isopropanol 5 %).

### Exemple 137 : 3-(aminoéthyl)-1-(cyclopropyl méthyl) indole

**Formule (II) :** R₁ = H ; n = 1 ; R₂ = cyclopropyl ; R₃ = R₄ = H

Huile brune purifiée par chromatographie sur gel de silice (éluant chloroforme 95 % / isopropylamine 5 %).

### Exemple 138 : 3-(2-aminoéthyl)-1-(2,5-diméthylbenzyl)-5-thiométhyl indole

**Formule (II) :** R₁ = 5-SCH₃ ; n = 1 ; R₂ = 2,5-diméthylphényl ; R₃ = R₄ = H

Huile brune purifiée par chromatographie sur gel de silice (éluant chloroforme 95 % / isopropylamine 5 %).

Selon le mode opératoire de l'exemple 17, les nitrovinyl indoles des exemples 139 à 143 ont été préparés.

### Exemple 139 : 1-(2,5-diméthylbenzyl)-2-méthyl-3-(2-nitrovinyl) indole

**Formule (IX) :** R₁ = 2-CH₃ ; n = 1 ; R₂ = 2,5-diméthylphényl ; R₃ = R₄ = H
Solide orange utilisé tel quel à l'étape suivante.
Point de fusion : 180°C.

### Exemple 140 : 1-(cyclopentyl)-2-méthyl-3-(2-nitrovinyl) indole

**Formule (IX) :** R₁ = 2-CH₃ ; n = 0 ; R₂ = cyclopentyl ; R₃ = R₄ = H

Huile orange purifiée par chromatographie sur gel de silice (éluant chloroforme).

### Exemple 141 : 2-phényl-3-(2-nitrovinyl) indole

**Formule (IX) :** R₁ = 2-phényl ; n = 0 ; R₂ = H ; R₃ = R₄ = H
Solide orange utilisé tel quel à l'étape suivante.
Point de fusion : 180°C.
Selon le mode opératoire de l'exemple 17 mais en utilisant le nitroéthane au lieu du nitrométhane on obtient les composés des exemples 142 et 143.

### Exemple 142 : 1-(2,5-diméthylbenzyl)-3-(2-méthyl-2-nitrovinyl) indole

**Formule (IX) :** R₁ = H ; n = 1; R₂ = 2,5-diméthylphényl ; R₃ = H ; R₄ = CH₃
Solide jaune cristallisé dans l'eau.
Point de fusion : 160°C.

### Exemple 143 : 1-cyclopentyl-3-(2-méthyl-2-nitrovinyl) indole

**Formule (IX) :** R₁ = H ; n = 0 ; R₂ = cyclopentyl ; R₃ = H ; R₄ = CH₃

Solide jaune cristallisé dans l'isopropanol.
Point de fusion: 135°C.
Selon le procédé de l'exemple 5 et à partir de 3-formyl indoles substitués convenablement les produits suivants des exemples 144 à 146 ont été préparés :

### Exemple 144 : 1-(2,5-diméthylbenzyl) 3-formyl-2-méthyl indole

**Formule (VIII) :** R₁ = 2-CH₃ ; n = 1 ; R₂ = 2,5-diméthylphényl
Solide jaune cristallisé dans l'éther, utilisé tel quel à l'étape suivante.
Point de fusion : 155°C.

### Exemple 145 : 1-(cyclopentyl)-3-formyl-2-méthyl indole

**Formule (VIII) :** R₁ = 2-CH₃ ; n = 0 ; R₂ = cyclopentyl

Huile brune purifiée par chromatographie sur gel de silice (éluant chloroforme 95 % / méthanol 5 %).

### Exemple 146 : 3-formyl-2-phényl indole

**Formule (VIII) :** R₁ = 2-phényl ; n = 0 ; R₂ = H

Solide crème utilisé tel quel à l'étape suivante, obtenu selon le mode opératoire décrit dans le J. Med. Chem. (1964), 7, 735.
Point de fusion : 253°C.
Selon le mode opératoire de l'exemple 1, mais en utilisant le méthoxy-2 éthylamine le composé suivant de l'exemple 147 a été préparé.

### Exemple 147 : β-D-Ribofuranuronamide, 1-(6-chloro -9H-purin-9-yl)-1-deoxy-N-(2-méthoxyéthyl)-2,3-O-(1-méthyléthylidène)

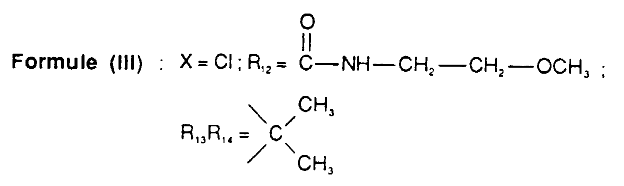

Huile brune purifiée par chromatographie sur gel de silice (éluant chloroforme 95 %/ méthanol 5 %).
Selon le mode opératoire de l'exemple 46 et en utilisant l'uronamide de l'exemple 2, le composé de l'exemple 148 a été préparé sous forme de solide amorphe.

### Exemple 148 : β-D-Ribofuranuronamide, 1-deoxy-1- [6-[[2-[1-(2,5-diméthylbenzyl) indol-3-yl] éthyl] amino]-9H-purin-9-yl]-N-éthyl-2,3-O-(1-méthyléthylidène)

**Formule (IV) :** R₁ = H ; n = 1 ; R₂ = 2,5-diméthylphényle ; R₃ = R₄ = H ; 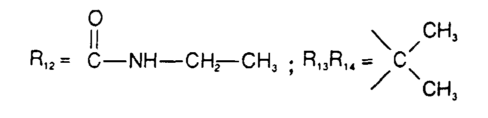 Selon le procédé de l'exemple 61, le composé de l'exemple 149 a été préparé.

### Exemple 149 : β-D-Ribofuranuronamide, 1-deoxy-1- [6-[[2-[1-(2,5-diméthylbenzyl) indol-3-yl] éthyl] amino]-9H-purin-9-yl]-N-éthyl

**Formule (I) :** R₁ = H ; n = 1 ; R₂ = 2,5-diméthylphényl ; R₃ = R₄ = H ; R₅ = -NH-CH₂-CH₃ Formule brute : C₃₁H₃₅N₇O₄, 0.4 H₂O
Point de fusion : 133°C

### PHARMACOLOGIE

L'activité pharmacologique des produits des exemples a été évaluée selon deux approches distinctes : fixation sur les récepteurs à l'adénosine et/ou mise en évidence d'une activité analgésique par le test à la phénylbenzoquinone.

### I Mode opératoire

### 1· Fixation sur les récepteurs à l'adénosine

### Principe

L'affinité des produits des exemples pour les récepteurs adénosinergiques A₁ et A₂ centraux de rat est déterminée par la technique de compétition à l'aide d'un ligand radioactif fixé spécifiquement, soit sur les récepteurs A₁ ([³H] PIA), soit sur les récepteurs A₂ ([³H] NECA)

### Méthode

### · Méthode d'étude des récepteurs A₁

### - Préparation membranaire

Après sacrifice de l'animal par décapitation, le cerveau est rapidement prélevé et lavé dans du sérum physiologique froid. Les deux hémisphères sont séparés, pesés et chacun d'eux est introduit dans un tube polyallomer contenant 25 volumes de tampon d'homogénéïsation froid. L'homogénéïsation est réalisée à l'aide d'un Ultra-Turrax durant 30 secondes (3 fois 10 secondes espacées de 10 secondes, 70 % de la vitesse maximale). Le broyat obtenu est centrifugé à 1000 g (≈ 3000 rpm) pendant 10 minutes à 4°C.

Le surnageant est centrifugé à nouveau à 48000 g (≈ 20000 rpm) durant 20 minutes à 4°C.

Au terme de cette étape, le culot est repris par 4 volumes de tampon d'homogénéïsation, remis en suspension à l'aide d'un vortex et homogénéïsé avec l'Ultra-Turrax. L'adénosine déaminase est alors ajoutée à raison de 1 U/ml, soit 1 µl/ml d'homogénat, en utilisant une seringue Hamilton de 10 µl.

Ainsi traité, l'homogénat est agité pendant 30 minutes à température ambiante ; puis il est centrifugé à 80000 g (≈ 20000 rpm) durant 30 minutes à 4°C.

Le culot obtenu est remis en suspension dans 10 volumes de tampon d'homogénéïsation et passé à l'Ultra-Turrax pendant 20 secondes (2 fois 10 secondes espacées de 10 secondes, 70 % de la vitesse maximale).

L'homogénat ainsi préparé est utilisé pour les essais de compétition. Il est conservé à 4°C si les études ont lieu dans la journée, ou stocké à -20°C sous forme d'aliquotes de 10 ml.

### - Essai de compétition

Après avoir décongelé l'homogénat à température ambiante, celui-ci est passé au Potter (6 allers-retours manuels, vitesse 6) dilué au 2/5 dans le tampon d'incubation et placé dans le bain marie thermostaté à 4°C sous agitation jusqu'à la fin de l'expérimentation.

50 µl de [³H] PIA à 100 nM, soit 2.5 nM, dans le milieu réactionnel final en tenant compte de la dilution au 1/40, et 50 µl de produit de l'exemple aux concentrations envisagées (10⁻⁵ M et 10⁻⁷ M) sont introduits dans les tubes réactionnels. La réaction est déclenchée par l'ajout de 1 ml d'homogénat et 900 µl de tampon d'incubation. Pour tous les bêta-bloqueurs étudiés, la procédure est identique.

Les tubes sont agités et incubés au bain-marie à 20°C pendant 30 minutes. Au terme de l'incubation, les tubes sont filtrés sur du papier Whatman GF/B. Chaque tube est lavé deux fois avec 2 ml de tampon de rinçage, puis les filtres eux-mêmes sont rincés avec 3 ml de ce même tampon.

Les filtres sont alors transférés dans des fioles de comptage et 10 ml de liquide scintillant (Ready Solv HP/b, Beckman) sont ajoutés.

Les fioles sont stockées au réfrigérateur durant une nuit après les avoir agitées, puis la radioactivité est déterminée dans un compteur à scintillation liquide.

Pour chaque concentration étudiée, 3 essais sont effectués. La fixation non spécifique du [³H] PIA est appréciée en mesurant la quantité de radioactivité retenue sur le filtre en présence de 10⁻⁵ M de phénylisopropyladénosine (PIA). La valeur de la fixation non spécifique est systématiquement soustraite à celle des essais.

### · Méthode d'étude des récepteurs A₂

### - Préparation membranaire

Après décapitation de l'animal, le cerveau est rapidement prélevé et lavé dans du sérum physiologique froid. Les deux hémisphères sont séparés et sur chacun d'eux, le striatum est prélevé (Bruns et al., 1986), pesé et introduit dans un tube polyallomer contenant 10 volumes de tampon d'homogénéïsation froid. Le tissu est homogénéïsé avec un Ultra-Turrax durant 30 secondes (3 fois 10 secondes espacées de 10 secondes, 70 % de la vitesse maximale). Le broyat est centrifugé à 50000 g (≈ 20500 rpm) durant 10 minutes à 4°C.

Le culot obtenu est remis en suspension dans 10 volumes de tampon d'homogénéïsation à l'aide d'un vortex et homogénéïsé avec l'Ultra-Turrax (5 à 10 secondes, 70 % de la vitesse maximale).

L'adénosine déaminase est alors ajoutée à raison de 1 U/ml, soit 1 µl/ml d'homogénat, en utilisant une seringue Hamilton 10 µl. L'homogénat ainsi traité est agité à température ambiante pendant 30 minutes.

Au terme de l'incubation, celui-ci est centrifugé à 50000 g (≈ 20500 rpm) durant 10 minutes à 4°C.

Le culot est repris par 5 volumes de tampon d'homogénéisation froid, passé à l'Ultra-Turrax (2 fois 10 secondes espacées de 10 secondes, 70 % de la vitesse maximale) et l'homogénat ainsi préparé est finalement congelé à -70°C.

### - Essai de compétition

Après avoir décongélé l'homogénat à température ambiante, 15 volumes de tampon d'incubation sont ajoutés. L'homogénat est agité au vortex, passé au Potter (6 allers-retours, vitesse 6), dilué au 1/10 dans le tampon d'incubation et finalement placé dans le bain-marie thermostaté à 4°C sous agitation jusqu'à la fin de l'expérimentation.

50 µl de [³H] NECA à 160nM, soit 4 nM, dans le milieu réactionnel final en tenant compte de la dilution au 1/40, et 50 µl de produit de l'exemple aux concentrations envisagées (10⁻⁵ M et 10⁻⁷ M) sont introduits dans les tubes réactionnels. La réaction est déclenchée par l'ajout de 1 ml d'homogénat et 900 µl de tampon d'incubation. Pour tous les composés étudiés, la procédure est similaire.

Les tubes sont agités et incubés au bain-marie à 25°C pendant 60 minutes. Au terme de l'incubation, les tubes sont filtrés sur du papier Whatman GF/B. Chaque tube est lavé deux fois avec 2 ml de tampon de rinçage, puis les filtres eux-mêmes sont rincés avec 3 ml de ce même tampon avant d'être transférés dans des fioles de comptage.

10 ml de liquide scintillant (Ready Solv HP/b, Beckman) sont ajoutés dans toutes les fioles. Celles-ci sont agitées et stockées au réfrigérateur durant une nuit. La radioactivité est déterminée dans un compteur à scintillation liquide.

Pour chaque concentration étudiée, 3 essais sont effectués. La fixation non spécifique du [³H] NECA est déterminée en mesurant la quantité de radioactivité retenue sur le filtre en présence de 5 µM de N-éthylcarboxamido-adénosine (NECA). La valeur de la fixation non spécifique est systématiquement déduite de celle des essais.

### ·. Traitement des données

Les résultats sont exprimés par produit sous forme de pourcentage (n = 3) de déplacement du radioligand marqué aux concentrations de 10⁻⁵ M et 10⁻⁷ M.

### 2· Test à la phénylbenzoquinone

### Méthode

L'injection intrapéritonéale de phénylbenzoquinone provoque chez la souris des mouvements de torsion et d'étirement. Les analgésiques préviennent ou diminuent ce syndrome qui peut être considéré comme l'extériorisation d'une douleur abdominale diffuse.

La solution de phénylbenzoquinone à 0.02 % dans l'eau est administrée sous un volume de 1 ml/100 g.

Les produits des exemples sont administrés par voie orale une heure avant l'injection de phénylbenzoquinone.

Les étirements et torsions sont comptés pour chaque souris durant une période d'observation de 5 minutes.

### II Résultats

Les résultats des expériences mettent en évidence l'affinité des produits des exemples pour les récepteurs à l'adénosine et leurs propriétés antalgiques sont respectivement présentées dans les tableaux 1 et 2.

**tableau 1**

| Produit de l'exemple | % de déplacement du ligand marqué | | | |
|---|---|---|---|---|
| | A1 | | A2 | |
| | 1E-5M | 1E-7M | 1E-5M | 1E-7M |
| Exemple 61 | 98 | 47 | 91 | 30 |
| | | | | |
| Exemple 63 | 98 | 61 | 90 | 19 |
| | | | | |
| Exemple 64 | 96 | 21 | 90 | 28 |
| | | | | |
| Exemple 65 | 87 | 4 | 91 | 14 |
| | | | | |
| Exemple 66 | 97 | 37 | 89 | 21 |
| | | | | |
| Exemple 67 | 96 | 29 | 83 | 13 |
| | | | | |
| Exemple 68 | 93 | 26 | 84 | 9 |
| | | | | |
| Exemple 69 | 93 | 63 | 90 | 12 |
| | | | | |
| Exemple 70 | 100 | 91 | 94 | 44 |
| | | | | |
| Exemple 71 (citrate) | 99 | 91 | 96 | 44 |
| | | | | |
| Exemple 71 (base) | 99 | 94 | 92 | 46 |
| | | | | |
| Exemple 85 | 100 | 50 | 71 | 3 |
| | | | | |
| Exemple 86 | 100 | 16 | 91 | 17 |
| | | | | |
| Exemple 87 | 56 | 5 | 60 | 7 |
| | | | | |
| Exemple 92 | 96 | 21 | 84 | 9 |
| | | | | |
| Exemple 93 | 96 | 45 | 85 | 22 |

**tableau 2**

| | **Test à la phénylbenzoquinone** |
|---|---|
| Produits de l'exemple | Dose inhibitrice 50 mg/kg V.O. |
| Exemple 61 | 0.90 |
| | |
| Exemple 63 | 10 |
| | |
| Exemple 64 | 0.3 |
| | |
| Exemple 65 | 0.3 |
| | |
| Exemple 66 | 3 |
| | |
| Exemple 67 | 3 |
| | |
| Exemple 68 | 2.4 |
| | |
| Exemple 71 (citrate) | 7.3 |
| | |
| Exemple 85 | 0.9 |
| | |
| Exemple 86 | ≈ 60 |
| | |
| Exemple 87 | 1.2 |
| | |
| Exemple 92 | 3.6 |
| | |
| Exemple 93 | 2 |

### III Toxicologie

La tolérance des produits des exemples a été appréciée chez le rat après administration par voie orale. Celle-ci s'est révélée bonne jusqu'à la dose de 100 mg/kg.

### IV Conclusion

Les produits des exemples décrits dans la présente invention possédent des propriétés analgésiques particulièrement intéressantes dont le mécanisme d'action original résulte d'une interaction avec les récepteurs à l'adénosine.

## Revendications

1. Dérivés d'adénosine caractérisés en ce qu'ils répondent à la formule générale (I) : 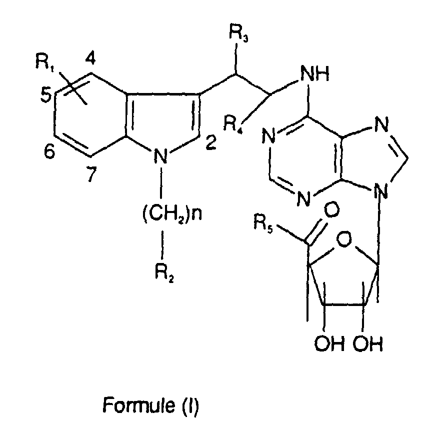 dans laquelle :
- R₁ représente un atome d'hydrogène, un atome d'halogène, un radical alkyle inférieur, un radical O-alkyle inférieur, un radical S-alkyle inférieur, ou un radical phényle et peut se trouver en position 2,4,5,6 ou 7 de l'indole,
- n est un nombre entier de 0 à 4,
- R₂ représente un radical alkyle inférieur, un radical alcényle inférieur, un radical alcynyle inférieur, un radical cycloalkyle en C₃-C₇, un radical O-alkyle inférieur,
- un radical phényle ou naphtyle non substitué ou substitué par un à quatre substituants identiques ou différents choisis parmi un atome d'halogène, un groupement nitro, alkyle inférieur, O-alkyle inférieur, S- alkyle inférieur, NR₇R₈, R₇ et R₈ représentant l'atome d'hydrogène ou un radical alkyle inférieur,
- un radical hétérocyclique choisi parmi la pyridine, le thiophène non substitué ou substitué par un à quatre substituants identiques ou différents choisis parmi un atome d'halogène, un groupement nitro, alkyle inférieur, O-alkyle inférieur, S-alkyle inférieur,
- ou encore quand n est égal à 2,3 ou 4 un groupement -NR₉R₁₀, R₉ et R₁₀ représentant simultanément un radical alkyle inférieur ou formant ensemble avec l'atome d'azote auquel ils sont rattachés un hétérocycle choisi parmi la morpholine, la pipéridine, la pyrrolidine,
- R₃ et R₄ identiques ou différents représentent l'atome d'hydrogène ou un radical alkyle inférieur,
- R₅ représente un groupement NHR₁₁, R₁₁ étant un radical alkyle inférieur, un radical cycloalkyle en C₃-C₇, une chaîne alkyle inférieur possédant une fonction alcool, éther ou encore un groupement -(CH₂)ₙ-NR₉R₁₀, n, R₉ et R₁₀ étant définis comme précédemment.

2. Dérivés de formule générale (I) selon la revendication 1, caractérisés en ce que :
- R₁ représente un atome d'hydrogène, un atome d'halogène, un radical alkyle inférieur, un radical O-alkyle inférieur, un radical S-alkyle inférieur, ou un radical phényle et peut se trouver en position 2 ou 5 de l'indole ;
- n est un nombre entier égal à 0, 1 ou 2 ;
- R₂ représente un radical alkyle inférieur, un radical alcényle inférieur, un radical alcynyle inférieur, un radical cycloalkyle en C₃-C₇, un radical O-alkyle inférieur ;
- un radical phényle ou naphtyle, non substitué ou substitué par un ou deux substituants identiques ou différents choisis parmi un atome d'halogène, un groupe nitro, alkyle inférieur, O-alkyle inférieur, -NR₇R₈, R₇ et R₈ représentant l'atome d'hydrogène ou un radical alkyle inférieur ;
- un radical hétérocyclique choisi parmi la pyridine, le thiophène non substitué ou substitué par un atome d'halogène ;
- ou encore quand n = 2, un groupement -NR₉R₁₀, R₉ et R₁₀ représentant simultanément un radical alkyle inférieur ou formant ensemble avec l'atome d'azote auquel ils sont attachés un hétérocycle choisi parmi la morpholine, la pipéridine, la pyrrolidine ;
- R₃ et R₄, identiques ou différents représentent l'atome d'hydrogène ou un radical alkyle inférieur ;
- R₅ représente un groupement NHR₁₁, R₁₁ étant un radical alkyle inférieur, un radical cycloalkyle en C₃-C₇, une chaîne alkyle inférieure possédant une fonction alcool ou éther.

3. Dérivés selon la revendication 1 ou 2 caractérisés en ce que R₁ représente l'atome d'hydrogène ou un radical choisi parmi méthyle ou méthoxy.

4. Dérivés selon la revendication 1 ou 2 caractérisés en ce que n représente un nombre choisi parmi 0,1 ou 2.

5. Dérivés selon la revendication 1 ou 2 caractérisés en ce que R₂ représente un radical choisi parmi méthoxy, cyclopentane, isopropyle, 2,5- diméthylphényl, pipéridine.

6. Dérivés selon la revendication 1 ou 2 caractérisés en ce que R₃ représente l'atome d'hydrogène.

7. Dérivés selon la revendication 1 ou 2 caractérisés en ce que R₄ représente l'atome d'hydrogène ou un radical méthyle.

8. Dérivés selon la revendication 1 ou 2 caractérisés en ce que R₅ représente un radical N-cyclopropyl amine.

9. Dérivés selon la revendication 1 ou 2 caractérisés en ce qu'ils sont choisis parmi les dérivés suivants :

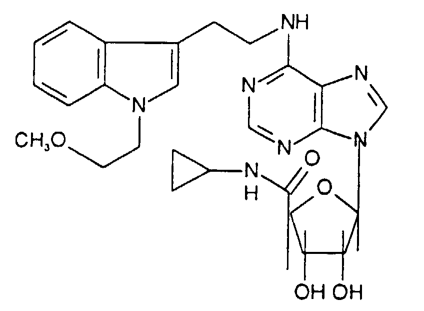

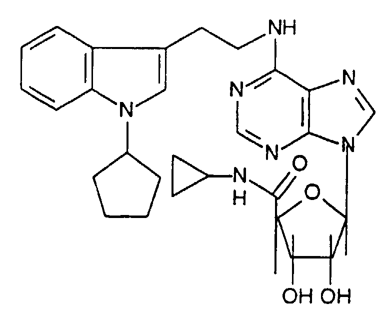

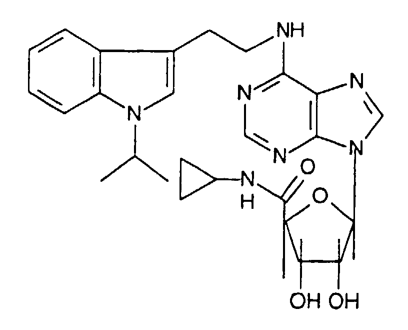

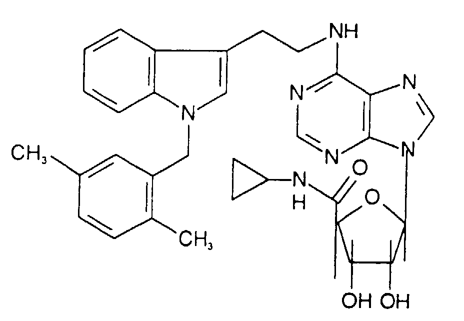

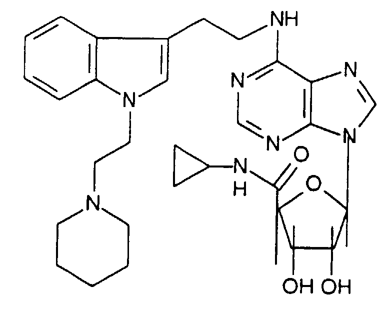

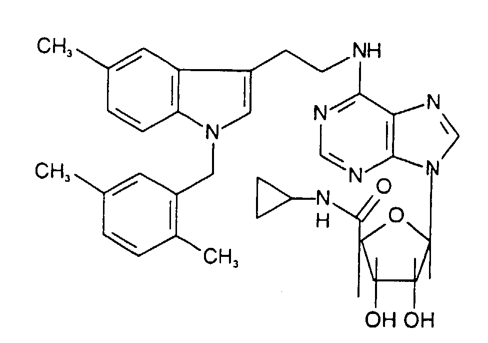

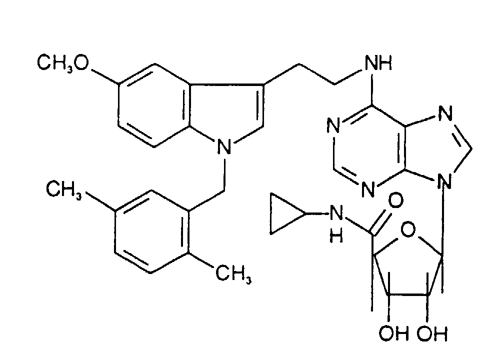

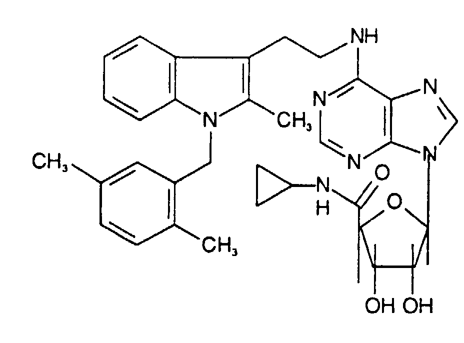

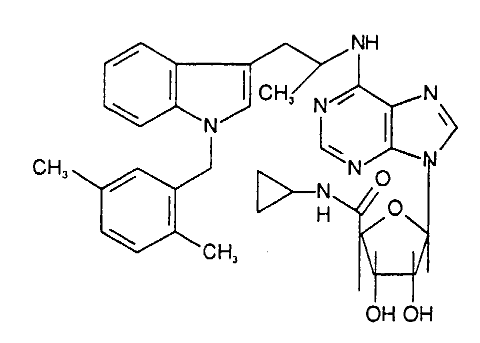

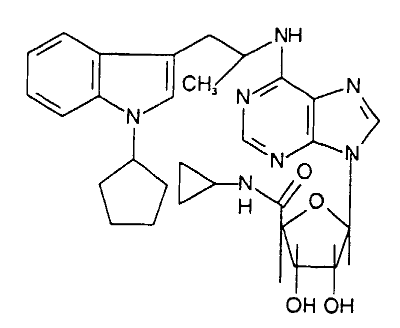

10. Procédés de préparation des composés de formule (I) selon l'une quelconque des revendications 1 à 9 caractérisés en ce qu'on fait réagir une amine de formule 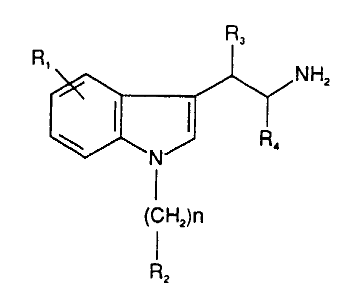 dans laquelle R₁, R₂, R₃, R₄ et n sont tels que définis à la revendication 1 ou 2 sur les 6-halopurines ribosides de formule 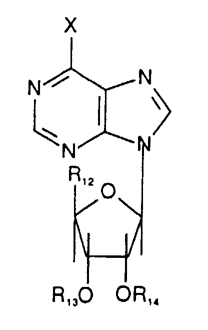 dans laquelle, X représente un atome d'halogène, R₁₂ représente un groupement COR₅, R₅ étant tel que défini à la revendication 1 ou 2 ou le groupement CH₂OH, R₁₃ et R₁₄ sont des groupements protecteurs comme acétyl, benzoyl ou benzyl par exemple ou pouvant former ensemble un autre groupement protecteur de structure dioxolane par exemple, dans un solvant comme un alcool ou le diméthylformamide en présence d'une base comme la triéthylamine, la pyridine ou un carbonate de sodium, potassium ou calcium ou encore en présence de deux équivalents de l'amine à une température comprise entre 20° et 140°C, la déprotection des alcools sera effectuée ensuite selon diverses méthodes connues de l'homme de l'art : en milieu basique avec une solution d'alcool ammoniacal ou en milieu acide avec une solution d'acide chlorhydrique normale ou d'acide formique à une température variant de 0° à 70°C selon la nature des groupements protecteurs.

11. Composition pharmaceutique, caractérisée en ce qu'elle comprend une quantité pharmaceutiquement efficace d'au moins un composé de formule (I) tel que défini à l'une quelconque des revendications 1 à 9, ou un de ses sels d'addition pharmaceutiquement acceptable, éventuellement incorporé dans un excipient, véhicule ou support pharmaceutiquement acceptable.

12. Composition pharmaceutique à activité antalgique caractérisée en ce qu'elle renferme une quantité pharmaceutiquement efficace d'au moins un composé de formule (I) tel que défini à l'une quelconque des revendications 1 à 9, ou un de ses sels d'addition pharmaceutiquement acceptable, éventuellement incorporé dans un excipient, véhicule ou support pharmaceutiquement acceptable.

13. Composition pharmaceutique à activité antihypertensive caractérisée en ce qu'elle renferme une quantité pharmaceutiquement efficace d'au moins un composé de formule (I) tel que défini à l'une quelconque des revendications 1 à 9, ou un de ses sels d'addition pharmaceutiquement acceptable, éventuellement incorporé dans un excipient, véhicule ou support pharmaceutiquement acceptable.

14. Procédé de préparation d'une composition pharmaceutique, caractérisé en ce qu'on incorpore une quantité pharmaceutiquement efficace d'au moins un composé de formule (I) tel que défini dans l'une quelconque des revendications 1 à 9 ou un de ses sels d'addition pharmaceutiquement acceptable, dans un excipient, véhicule ou support pharmaceutiquement acceptable.

15. Procédé selon la revendication 14, caractérisé en ce que la composition pharmaceutique est formulée sous forme de gélules, de comprimés dosés de 5 à 300 mg ou sous forme de préparations injectables dosées de 0,1 à 100 mg.

## Patentansprüche

1. Adenosin-Derivate, dadurch gekennzeichnet, daß sie die allgemeine Formel (I) aufweisen: 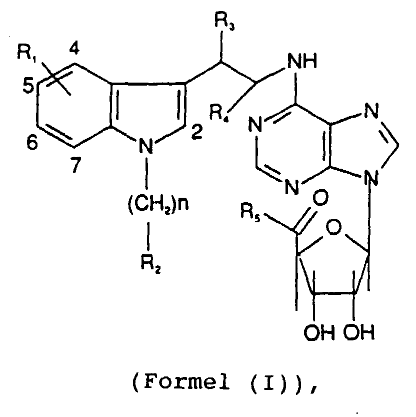 worin:
- R₁ ein Wasserstoffatom, ein Halogenatom, einen nied.Alkylrest, einen O-nied.Alkylrest, einen S-nied.Alkylrest oder einen Phenylrest bedeutet und sich in Stellung 2, 4, 5, 6 oder 7 des Indols befinden kann;
- n eine ganze Zahl von Null bis 4 ist;
- R₂ darstellt: einen nied.Alkylrest, einen nied.Alkenylrest, einen nied.Alkinylrest, einen C₃-C₇-Cycloalkylrest, einen O-nied.Alkylrest,
- einen Phenyl- oder Naphthylrest, unsubstituiert oder substituiert durch 1 bis 4 identische oder verschiedene Substituenten, ausgewählt aus einem Halogenatom, einer Nitro-, nied.Alkyl-, O-nied.Alkyl-, S-nied.Alkyl-Gruppe, NR₇R₈, wobei R₇ und R₈ ein Wasserstoffatom oder einen nied.Alkylrest bedeuten,
- einen heterocyclischen Rest, ausgewählt aus Pyridin, Thiophen, unsubstituiert oder substituiert durch 1 bis 4 identische oder verschiedene Substituenten, ausgewählt aus einem Halogenatom, einer Nitro-, nied.Alkyl-, O-nied.Alkyl-, S-nied.Alkyl-Gruppe,
- oder auch, wenn n 2, 3 oder 4 ist, eine Gruppe -NR₉R₁₀, wobei R₉ und R₁₀ gleichzeitig einen nied.Alkylrest darstellen oder gemeinsam mit dem Stickstoffatom, an das sie gebunden sind, einen Heterocyclus bilden, ausgewählt aus Morpholin, Piperidin, Pyrrolidin,
- R₃ und R₄, die gleich oder verschieden sind, ein Wasserstoffatom oder einen nied.Alkylrest bedeuten,
- R₅ eine Gruppe -NHR₁₁ darstellt, wobei R₁₁ ein nied.Alkylrest, ein C₃-C₇-Cycloalkylrest, eine nied.Alkylkette mit einer Alkohol-, Ether-Funktion oder auch eine Gruppe -(CH₂)ₙ-NR₉R₁₀ bedeutet, wobei n, R₉ und R₁₀ wie vorstehend definiert sind.

2. Derivate der allgemeinen Formel (I) nach Anspruch 1, dadurch gekennzeichnet, daß:
- R₁ ein Wasserstoffatom, ein Halogenatom, einen nied.Alkylrest, einen O-nied.Alkylrest, einen S-nied.Alkylrest oder einen Phenylrest bedeutet und sich in Stellung 2 oder 5 des Indols befinden kann;
- n eine ganze Zahl von Null, 1 oder 2 ist;
- R₂ darstellt: einen nied.Alkylrest, einen nied.Alkenylrest, einen nied.Alkinylrest, einen C₃-C₇-Cycloalkylrest, einen O-nied.Alkylrest,
- einen Phenyl- oder Naphthylrest, unsubstituiert oder substituiert durch 1 oder 2 identische oder verschiedene Substituenten, ausgewählt aus einem Halogenatom, einer Nitro-, nied.Alkyl-, O-nied.Alkyl-Gruppe, -NR₇R₈, wobei R₇ und R₈ ein Wasserstoffatom oder einen nied.Alkylrest bedeuten,
- einen heterocyclischen Rest, ausgewählt aus Pyridin, Thiophen, unsubstituiert oder substituiert durch ein Halogenatom,
- oder auch, wenn n = 2, eine Gruppe -NR₉R₁₀, wobei R₉ und R₁₀ gleichzeitig einen nied.Alkylrest darstellen oder gemeinsam mit dem Stickstoffatom, an das sie gebunden sind, einen Heterocyclus bilden, ausgewählt aus Morpholin, Piperidin, Pyrrolidin,
- R₃ und R₄, die gleich oder verschieden sind, ein Wasserstoffatom oder einen nied.Alkylrest bedeuten,
- R₅ eine Gruppe -NHR₁₁ darstellt, wobei R₁₁ ein nied.Alkylrest, ein C₃-C₇-Cycloalkylrest, eine nied.Alkylkette mit einer Alkohol- oder Ether-Funktion bedeutet.

3. Derivate nach Anspruch 1 oder 2, dadurch gekennzeichnet, daß R₁ ein Wasserstoffatom oder einen Rest, ausgewählt aus Methyl oder Methoxy, bedeutet.

4. Derivate nach Anspruch 1 oder 2, dadurch gekennzeichnet, daß n eine Zahl ausgewählt aus Null, 1 oder 2 ist.

5. Derivate nach Anspruch 1 oder 2, dadurch gekennzeichnet, daß R₂ einen Rest, ausgewählt aus Methoxy, Cyclopentan, Isopropyl, 2,5-Dimethylphenyl, Piperidin, darstellt.

6. Derivate nach Anspruch 1 oder 2, dadurch gekennzeichnet, daß R₃ ein Wasserstoffatom ist.

7. Derivate nach Anspruch 1 oder 2, dadurch gekennzeichnet, daß R₄ ein Wasserstoffatom oder einen Methylrest bedeutet.

8. Derivate nach Anspruch 1 oder 2, dadurch gekennzeichnet, daß R₅ ein N-Cyclopropylaminrest ist.

9. Derivate nach Anspruch 1 oder 2, dadurch gekennzeichnet, daß sie ausgewählt sind aus den folgenden Derivaten:

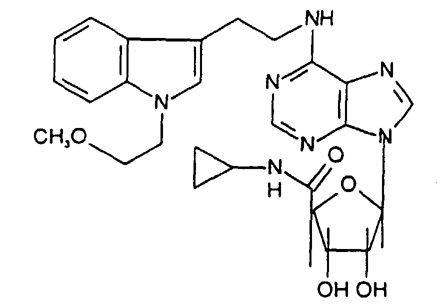

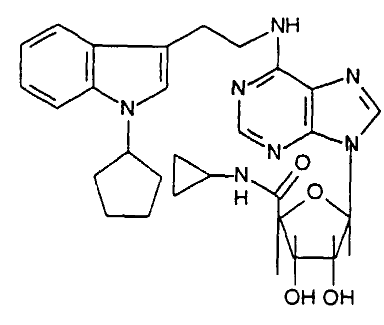

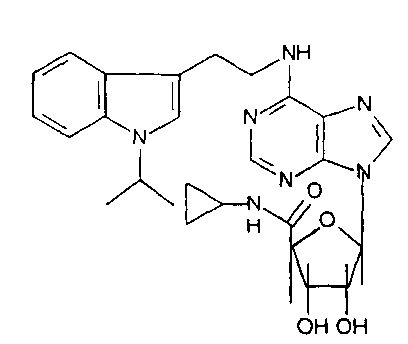

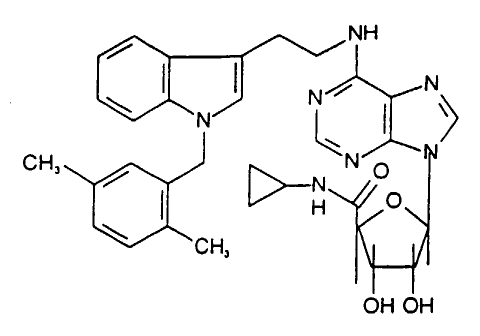

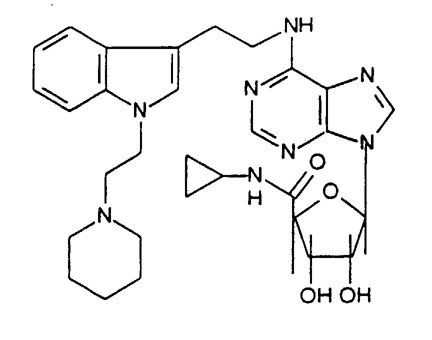

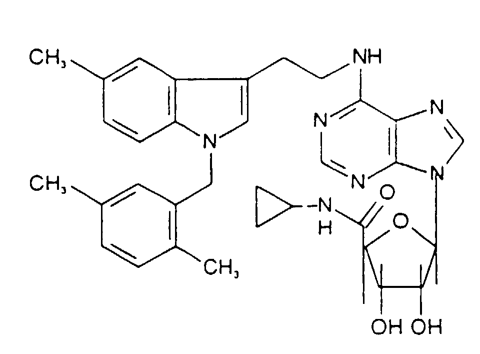

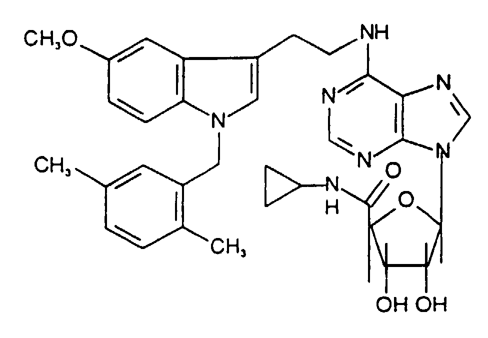

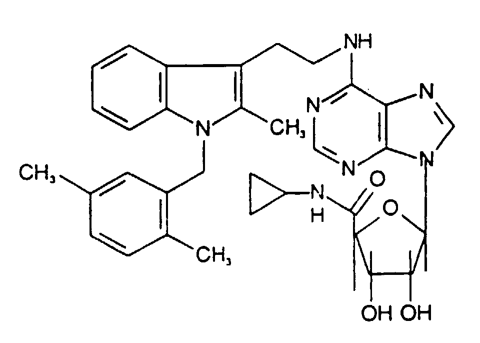

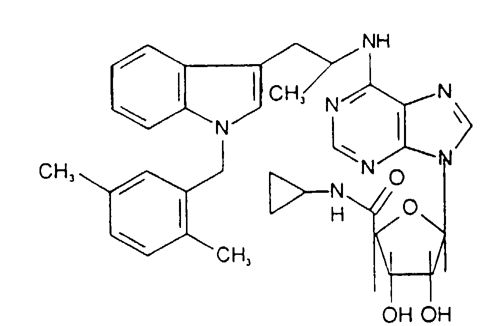

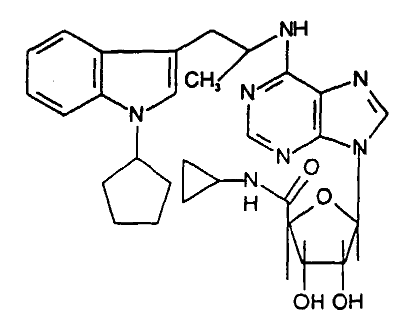

10. Verfahren zur Herstellung von Verbindungen der Formel (I) nach einem der Ansprüche 1 bis 9, dadurch gekennzeichnet, daß ein Amin der Formel 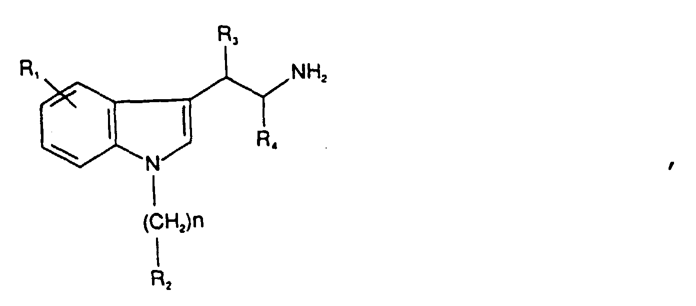 worin R₁, R₂, R₃, R₄ und n wie in Anspruch 1 oder 2 definiert sind, umgesetzt wird mit 6-Halogenpurinribosiden der Formel 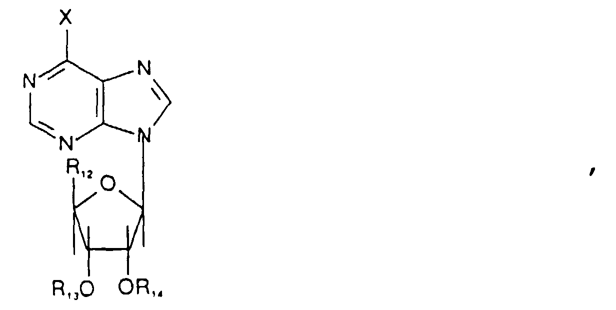 worin X ein Halogenatom bedeutet, R₁₂ ein Gruppe COR₅ darstellt, wobei R₅ wie in Anspruch 1 oder 2 definiert ist oder die Gruppe CH₂OH ist, R₁₃ und R₁₄ Schutzgruppen bedeuten, wie beispielsweise Acetyl, Benzoyl oder Benzyl, oder gemeinsam eine andere Schutzgruppe beispielsweise mit Dioxolan-Struktur bilden können, in einem Lösungsmittel, wie Alkohol oder Dimethylformamid in Anwesenheit einer Base, wie Triethylamin, Pyridin oder Natrium-, Kalium- oder Calciumcarbonat, oder auch in Anwesenheit von zwei Amin-Äquivalenten bei einer Temperatur zwischen 20°C und 140°C, anschließend das Entschützen der Alkohole gemäß verschiedenen Fachleuten bekannten Verfahren durchgeführt wird: in basischem Medium mit einer Ammoniak enthaltenden Alkohol-Lösung oder in saurem Medium mit einer normalen Salzsäure- oder Ameisensäure-Lösung bei einer Temperatur, die gemäß der Beschaffenheit der Schutzgruppen von 0°C bis 70°C variiert.

11. Pharmazeutische Zusammensetzung, dadurch gekennzeichnet, daß sie eine pharmazeutisch wirksame Menge zumindest einer Verbindung der Formel (I), wie in einem der Ansprüche 1 bis 9 definiert, oder eines ihrer pharmazeutisch annehmbaren Säureadditionssalze, gegebenenfalls eingeschlossen in einem pharmazeutisch annehmbaren Exzipienten, Vehikel oder Träger, umfaßt.

12. Pharmazeutische Zusammensetzung mit analgetischer Wirksamkeit, dadurch gekennzeichnet, daß sie eine pharmazeutisch wirksame Menge zumindest einer Verbindung der Formel (I), wie in einem der Ansprüche 1 bis 9 definiert, oder eines ihrer pharmazeutisch annehmbaren Säureadditionssalze, gegebenenfalls eingeschlossen in einem pharmazeutisch annehmbaren Exzipienten, Vehikel oder Träger, umfaßt.

13. Pharmazeutische Zusammensetzung mit antihypertensiver Wirksamkeit, dadurch gekennzeichnet, daß sie eine pharmazeutisch wirksame Menge zumindest einer Verbindung der Formel (I), wie in einem der Ansprüche 1 bis 9 definiert, oder eines ihrer pharmazeutisch annehmbaren Säureadditionssalze, gegebenenfalls eingeschlossen in einem pharmazeutisch annehmbaren Exzipienten, Vehikel oder Träger, umfaßt.

14. Verfahren zur Herstellung einer pharmazeutischen Zusammensetzung, dadurch gekennzeichnet, daß eine pharmazeutisch wirksame Menge zumindest einer Verbindung der Formel (I), wie in einem der Ansprüche 1 bis 9 definiert, oder eines ihrer pharmazeutisch annehmbaren Säureadditionssalze in einem pharmazeutisch annehmbaren Exzipienten, Vehikel oder Träger eingeschlossen wird.

15. Verfahren nach Anspruch 14, dadurch gekennzeichnet, daß die pharmazeutische Zusammensetzung in Form von Gelatinekapseln, mit 5 bis 300 mg dosierten Tabletten oder in Form von mit 0,1 bis 100 mg dosierten injizierbaren Zubereitungen formuliert wird.

## Claims

1. Adenosine derivatives characterised in that they are of general formula (I): 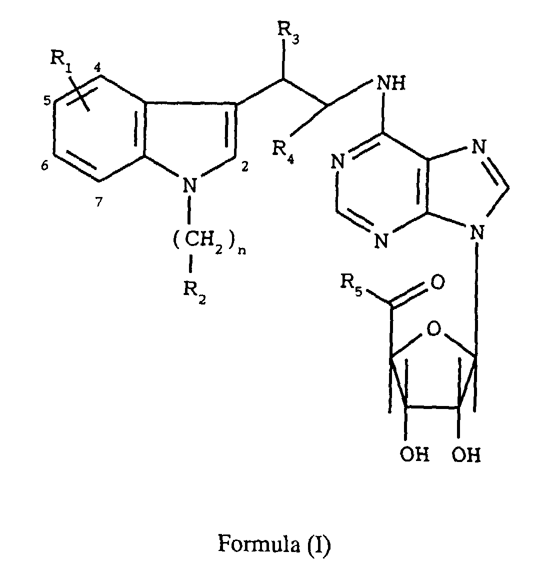 in which:
- R₁ represents a hydrogen atom, a halogen atom, a lower alkyl radical, a lower O-alkyl radical, a lower S-alkyl radical or a phenyl radical and can be located in the 2-, 4-, 5-, 6- or 7-position of the indole;
- n is an integer from 0 to 4;
- R₂ is a lower alkyl radical, a lower alkenyl radical, a lower alkynyl radical, a C₃-C₇-cycloalkyl radical or a lower O-alkyl radical;
- a phenyl or naphthyl radical which is unsubstituted or substituted by one to four identical or different substituents selected from a halogen atom, a nitro, lower alkyl, lower O-alkyl or lower S-alkyl group and a group -NR₇R₈, R₇ and R₈ being a hydrogen atom or a lower alkyl radical;
- a heterocyclic radical selected from pyridine and thiophene which is unsubstituted or substituted by one to four identical or different substituents selected from a halogen atom and a nitro, lower alkyl, lower O- alkyl or lower S-alkyl group;
- or even, when n is equal to 2, 3 or 4, a group -NR₉R₁₀, R₉ and R₁₀ simultaneously being a lower alkyl radical or forming, together with the nitrogen atom to which they are attached, a heterocycle selected from morpholine, piperidine and pyrrolidine;
- R₃ and R₄, which are identical or different, are a hydrogen atom or a lower alkyl radical; and
- R₅ is a group -NHR₁₁, R₁₁ being a lower alkyl radical, a C₃-C₇-cycloalkyl radical, a lower alkyl chain possessing an alcohol or ether functional group, or even a group -(CH₂)ₙ-NR₉R₁₀, n, R₉ and R₁₀ being as defined above.

2. Derivatives of general formula (I) according to claim 1, characterised in that:
- R₁ is a hydrogen atom, a halogen atom, a lower alkyl radical, a lower O-alkyl radical, a lower S-alkyl radical or a phenyl radical and can be located in the 2- or 5-position of the indole;
- n is an integer equal to 0, 1 or 2;
- R₂ is a lower alkyl radical, a lower alkenyl radical, a lower alkynyl radical, a C₃-C₇-cycloalkyl radical or a lower O-alkyl radical;
- a phenyl or naphthyl radical which is unsubstituted or substituted by one or two identical or different substituents selected from a halogen atom, a nitro, lower alkyl or lower O-alkyl group and a group -NR₇R₈, R₇ and R₈ being a hydrogen atom or a lower alkyl radical;
- a heterocyclic radical selected from pyridine and thiophene which is unsubstituted or substituted by a halogen atom;
- or even, when n = 2, a group -NR₉R₁₀, R₉ and R₁₀ simultaneously being a lower alkyl radical or forming, together with the nitrogen atom to which they are attached, a heterocycle selected from morpholine, piperidine and pyrrolidine;
- R₃ and R₄, which are identical or different, are a hydrogen atom or a lower alkyl radical; and
- R₅ is a group -NHR₁₁, R₁₁ being a lower alkyl radical, a C₃-C₇-cycloalkyl radical or a lower alkyl chain possessing an alcohol or ether functional group,

3. Derivatives according to claim 1 or claim 2 characterised in that R₁ is a hydrogen atom or a radical selected from methyl or methoxy.

4. Derivatives according to claim 1 or claim 2 characterised in that n is a number selected from 0, 1 or 2.

5. Derivatives according to claim 1 or claim 2 characterised in that R₂ is a radical selected from methoxy, cyclopentane, isopropyl, 2,5-dimethylphenyl and piperidine.

6. Derivatives according to claim 1 or claim 2 characterised in that R₃ is a hydrogen atom.

7. Derivatives according to claim 1 or claim 2 characterised in that R₄ is a hydrogen atom or a methyl radical.

8. Derivatives according to claim 1 or claim 2 characterised in that R₅ is an N-cyclopropylamine radical.

9. Derivatives according to claim 1 or claim 2 characterised in that they are selected from the following derivatives:

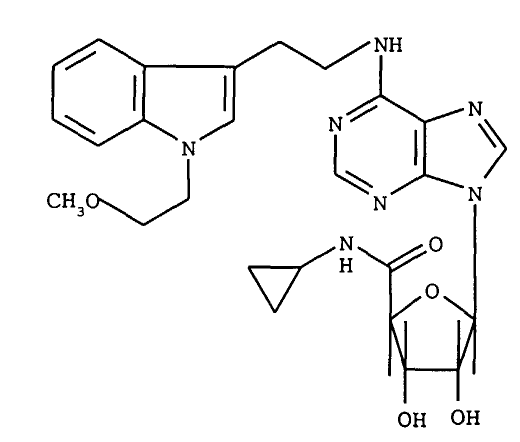

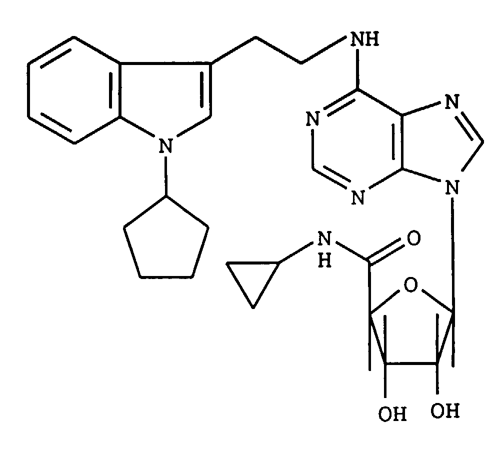

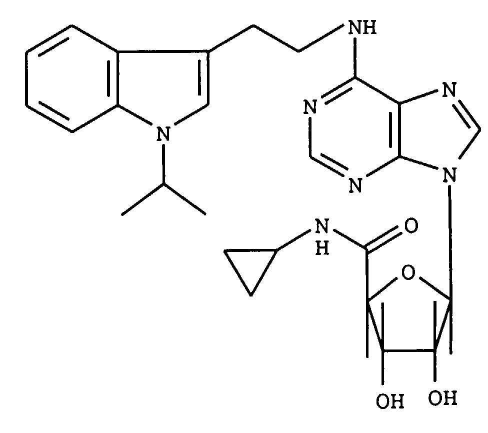

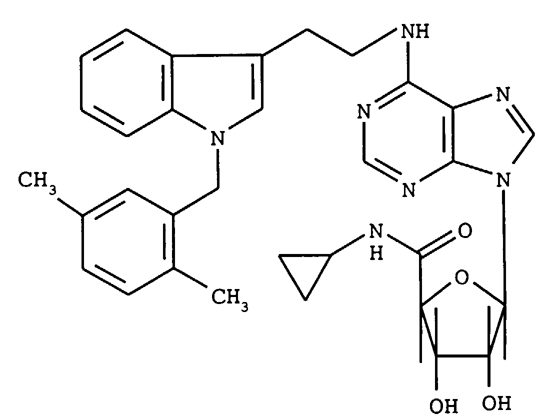

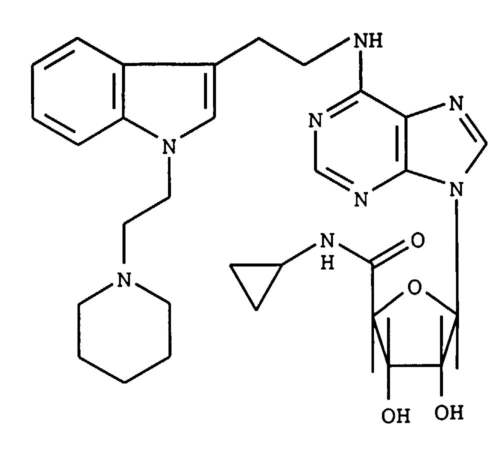

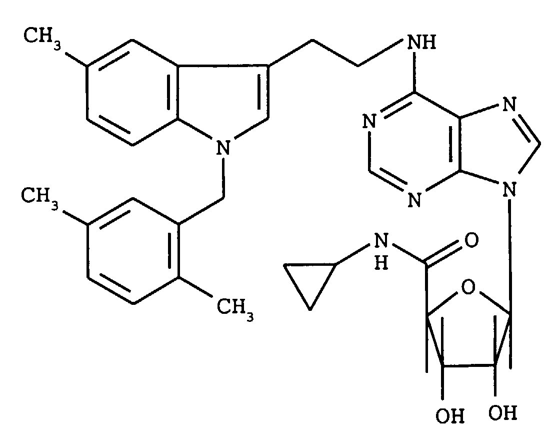

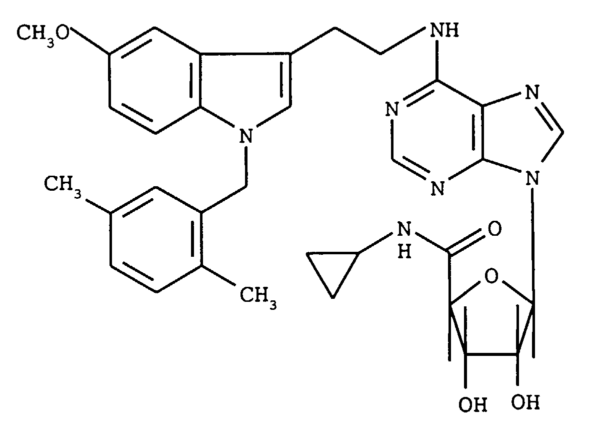

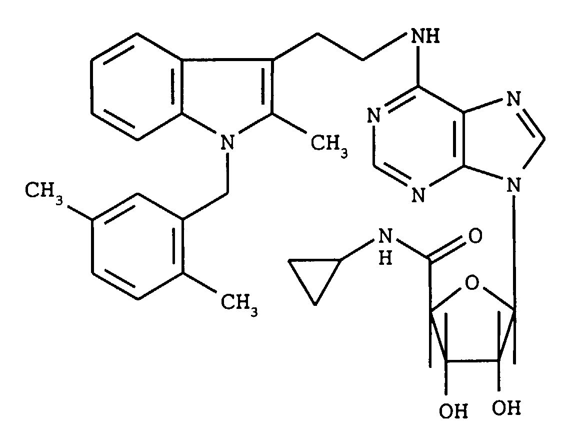

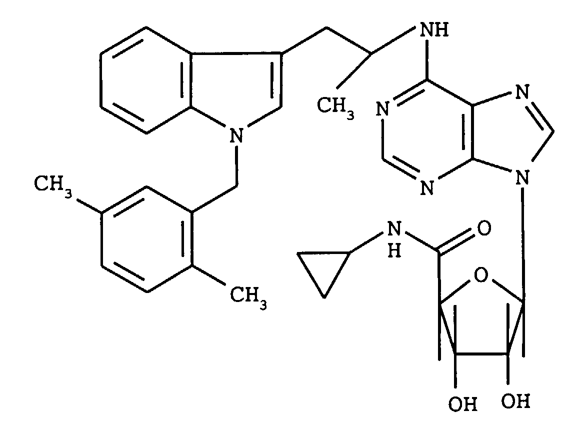

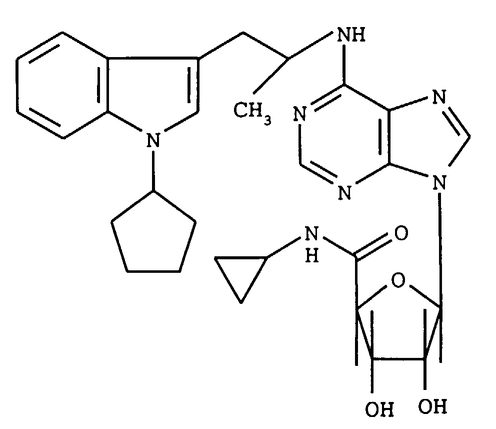

10. Methods of preparing the compounds of formula (I) according to any one of claims 1 to 9, characterised in that they comprise reacting an amine of the formula 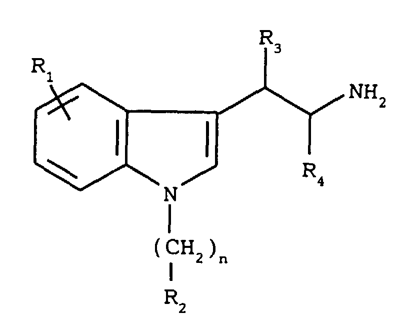 in which R₁, R₂, R₃, R₄ and n are as defined in claim 1 or claim 2, with the 6-halopurine ribosides of the formula 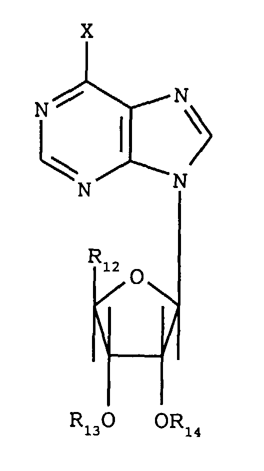 in which X is a halogen atom, R₁₂ is a group COR₅, R₅ being as defined in claim 1 or claim 2, or the CH₂OH group, and R₁₃ and R₁₄ are protecting groups such as, for example, acetyl, benzoyl or benzyl, or can together form another protecting group, for example of the dioxolan structure, in a solvent such as an alcohol or dimethylformamide, in the presence of a base such as triethylamine, pyridine or sodium, potassium or calcium carbonate, or even in the presence of two equivalents of the amine, at a temperature of between 20° and 140°C, after which the alcohols will be deprotected by various methods known to those skilled in the art, namely in a basic medium with an ammoniacal alcohol solution, or in an acid medium with a normal hydrochloric acid solution or a formic acid solution, at a temperature varying from 0° to 70°C depending on the nature of the protecting groups.

11. A pharmaceutical composition characterised in that it comprises a pharmaceutically effective amount of at least one compound of formula (I) as defined in any one of claims 1 to 9, or one of its pharmaceutically acceptable addition salts, which may or may not be incorporated in a pharmaceutically acceptable excipient, vehicle or carrier.

12. A pharmaceutical composition with analgesic activity, characterised in that it contains a pharmaceutically effective amount of at least one compound of formula (I) as defined in any one of claims 1 to 9, or one of its pharmaceutically acceptable addition salts, which may or may not be incorporated in a pharmaceutically acceptable excipient, vehicle or carrier.

13. A pharmaceutical composition with antihypertensive activity, characterised in that it contains a pharmaceutically effective amount of at least one compound of formula (I) as defined in any one of claims 1 to 9, or one of its pharmaceutically acceptable addition salts, which may or may not be incorporated in a pharmaceutically acceptable excipient, vehicle or carrier.

14. A method of preparing a pharmaceutical composition, characterised in that it comprises incorporating a pharmaceutically effective amount of at least one compound of formula (I) as defined in any one of claims 1 to 9, or one of its pharmaceutically acceptable addition salts, into a pharmaceutically acceptable excipient, vehicle or carrier.

15. A method according to claim 14, characterised in that the pharmaceutical composition is formulated as gelatine capsules or tablets containing from 5 to 300 mg of active ingredient, or as injectable preparations containing from 0.1 to 100 mg of active ingredient.
